# EUROPEAN PATENT APPLICATION

(11) **EP 2 039 779 A1**
(43) Date of publication of application: **25.03.2009**
(21) Application number: 07790920.8
(22) Date of filing: 18.07.2007
(51) Int. Cl.: C12Q 1/527, A61K 31/4245, A61K 31/662, A61K 45/00, A61P 1/16, A61P 1/18, A61P 3/10, A61P 7/06, A61P 21/00, A61P 25/00, A61P 29/00, A61P 43/00, C12N 9/96, C12N 15/09, G01N 33/15, G01N 33/50

(54) **SCREENING METHOD**

(30) Priority: 19.07.2006 JP 2006197483
(71) Applicant: Takeda Pharmaceutical Company Limited, Osaka-shi, Osaka 541-0045 (JP)
(72) Inventor: HAZAMA, Masatoshi c/oTAKEDA PHARMACEUTICAL COMPANY LIMITED, Osaka-shi, Osaka 540-8645 (JP); IWAKAMI, Norihisa c/oTAKEDA PHARMACEUTICAL COMPANY LIMITED, Osaka-shi Osaka 540-8645 (JP); YASHIRO, Hiroaki, Osaka-shi Osaka 532-8686 (JP)
(74) Representative: Brearley, Helen Rebecca
(86) International application number: PCT/JP2007/064165
(87) International publication number: WO 2008/010511

(57) **Abstract**

The present invention provides a method of screening for a therapeutic drug for diabetes or a nerve system disease, including using ferrochelatase, and a ferrochelatase activator containing a compound represented by the formula: 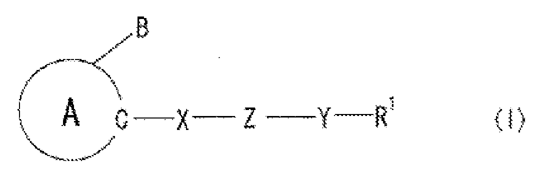 wherein ring A is a 5-membered aromatic heterocycle containing two or more nitrogen atoms and optionally further having substituent(s);
B is an optionally substituted hydrocarbon group or an optionally substituted heterocyclic group;
X is a divalent noncyclic hydrocarbon group;
Z is -O-, -S-, -NR²-, -CONR²- or -NR²CO- wherein R² is a hydrogen atom or an optionally substituted alkyl group;
Y is a bond or a divalent noncyclic hydrocarbon group; and
R¹ is an optionally substituted cyclic group, an optionally substituted amino group or an optionally substituted acyl group, or a salt thereof.

## Description

### Technical Field

The present invention relates to a screening method for a drug for the prophylaxis or treatment of diabetes or a nerve system disease. Specifically, the present invention relates to a screening method for a drug for the prophylaxis or treatment of diabetes or a nerve system disease, which comprises using ferrochelatase. The present invention moreover relates to a ferrochelatase activator and a pharmaceutical agent comprising a ferrochelatase activator.

### Background of the Invention

As one of the causes of diabetes or nerve system diseases, abnormality of mitochondria is known. For example, non-patent document 1 (Science (2005) 307, 384-387) describes that the abnormality of mitochondria could cause insulin resistance and dysfunction of pancreatic β cells in type 2 diabetes. In addition, non-patent document 2 (The Journal of Biological Chemistry (2001) 276(51), 48410-48416) and non-patent document 3 (PNAS (2002) 99(23), 14807-14812) have experimentally shown that lack of hem in mitochondria could be an important factor in the neurodegenerative process caused by aging and Alzheimer's disease.

Ferrochelatase is present in mitochondria, and is an enzyme that provides hem (i.e., iron-porphyrin complex) to various proteins by catalyzing coordination of iron in protoporphyrin IX (Cellular Molecular Life Sciences, vol. 57, p. 1909-26, 2000). In mitochondria, since proteins involved in electron transport chain such as cytochrome c oxidase and the like, catalase involved in radical treatment and the like require hem, ferrochelatase is considered to play an important role in mitochondrial function and intracellular redox control.

However, the relationship between ferrochelatase and diabetes or neurodegenerative diseases is not clear, and an approach of treating diabetes or nerve system diseases by activating ferrochelatase is not known.

Meanwhile, the compound represented by the formula (I) in the present specification is described as a compound having a neurotrophic factor production and secretion promoting action in patent document 1 (W02004/039365). However, it is not known that this compound has a ferrochelatase activating action.
patent document 1: W02004/039365
non-patent document 1: Science, vol. 307, p. 384-387, 2005
non-patent document 2: The Journal of Biological Chemistry, vol. 276, No. 51, p. 48410-48416, 2001
non-patent document 3: Proceedings of the National Academy of Sciences of the United States of America, vol. 99, No. 23, p. 14807-14812, 2002

### Disclosure of the Invention

### Problems to be Solved by the Invention

An object of the present invention is to provide an agent for the prophylaxis or treatment of diseases caused by abnormality (e.g., decrease in function) of mitochondria, and a means of developing such a prophylactic or therapeutic agent.

### Means of Solving the Problems

The present inventors have conducted intensive studies in an attempt to solve the above-mentioned problems and found a correlation between decreased mitochondrial function due to decreased ferrochelatase activity and diabetes, and further conducted intensive studies and completed the present invention.

Accordingly, the present invention relates to the following.
1) A method of screening for a therapeutic drug for diabetes or a nerve system disease, comprising using ferrochelatase.
2) The screening method of the above-mentioned 1), comprising selecting a substance that activates ferrochelatase.
3) A ferrochelatase activator comprising a compound represented by the formula: 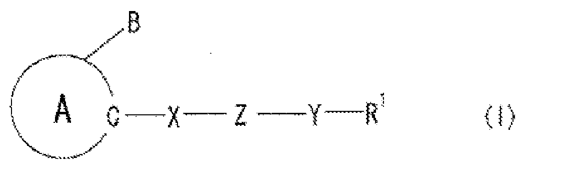 wherein ring A is a 5-membered aromatic heterocycle containing two or more nitrogen atoms and optionally further having substituent(s);
   B is an optionally substituted hydrocarbon group or an optionally substituted heterocyclic group;
   X is a divalent noncyclic hydrocarbon group;
   Z is -O-, -S-, -NR²-, -CONR²- or -NR²CO- wherein R² is a hydrogen atom or an optionally substituted alkyl group;
   Y is a bond or a divalent noncyclic hydrocarbon group; and
   R¹ is an optionally substituted cyclic group, an optionally substituted amino group or an optionally substituted acyl group, or a salt thereof.
4) An agent for the prophylaxis or treatment of diabetes or a nerve system disease, which is associated with a decreased mitochondrial function in a tissue, mitochondrial encephalomyopathy, fibromyalgia, pancreatic exhaustion or steatohepatitis, comprising a ferrochelatase activator.
5) An agent for the prophylaxis or treatment of anemia, comprising a ferrochelatase activator.
6) A method for the prophylaxis or treatment of diabetes or a nerve system disease, which is associated with a decreased mitochondrial function in a tissue, mitochondrial encephalomyopathy, fibromyalgia, pancreatic exhaustion or steatohepatitis in a mammal, comprising administering an effective amount of a ferrochelatase activator to the mammal.
7) A method for the prophylaxis or treatment of anemia in a mammal, comprising administering an effective amount of a ferrochelatase activator to the mammal.
8) Use of a ferrochelatase activator for the production of an agent for the prophylaxis or treatment of diabetes or a nerve system disease, which is associated with a decreased mitochondrial function in a tissue, mitochondrial encephalomyopathy, fibromyalgia, pancreatic exhaustion or steatohepatitis.
9) Use of a ferrochelatase activator for the production of an agent for the prophylaxis or treatment of anemia.

### Effect of the Invention

Using the screening method of the present invention, a pharmaceutical agent for the prophylaxis or treatment of a disease caused by abnormality of mitochondria can be developed. Specifically, a substance obtained by the screening method of the present invention is useful for the prophylaxis or treatment of a disease showing a decreased mitochondrial function in a tissue and a disease associated with a decrease in the hem in the body, since it can improve mitochondrial function by promoting the production of hem by activation of ferrochelatase present in mitochondria.

### Detailed Description of the Invention

The content of the present invention is explained in detail in the following.

The present invention provides a screening method of a drug for the prophylaxis or treatment of diabetes or a nerve system disease (hereinafter to be sometimes abbreviated as the screening method of the present invention). Specifically, the screening method of the present invention comprises selecting a substance that activates ferrochelatase. More specifically, the screening method of the present invention comprises
a) incubating ferrochelatase together with a porphyrin, which is a substrate thereof, and a divalent metal ion in a buffer both in the presence and absence of a test substance;
b) comparing a ferrochelatase activity in the presence of the test substance with a ferrochelatase activity in the absence of the test substance;
c) selecting a test substance that increases a ferrochelatase activity, compared to the ferrochelatase activity in the absence of the test substance, as a substance that activates ferrochelatase.

Examples of the porphyrins to be used for the above-mentioned screening method include protoporphyrin IX, deuteroporphyrin, mesoporphyrin and the like. Of these, protoporphyrin IX is preferable.

Examples of the divalent metal ion to be used for the above-mentioned screening method include divalent iron, zinc ion, cobalt ion and the like. Of these, divalent iron or zinc ion is preferable. These divalent metal ions may be used in the form of radioisotope as necessary.

Examples of the buffer to be used for the above-mentioned screening method include Tris-hydrochloric acid (Tris-HCl) buffer, phosphate buffer and the like. The pH of the buffer is generally 7.0 - 8.0, preferably 7.5 - 8.0.

Incubation is generally performed at about 20°C to 40°C for about 0.1 - 12 hr (preferably about 0.1 - 2 hr).

In the present specification, the activity of ferrochelatase means an activity of producing a porphyrin complex from a porphyrin and a divalent metal (e.g., an activity to produce protoheme from protoporphyrin IX and a divalent iron).

The activity of ferrochelatase can be measured, for example, using an amount of divalent metal ion incorporated in a porphyrin, a production amount of porphyrin complex and the like as an index.

Each index can be quantified according to a method known per se (see, for example, The Journal of Biological Chemistry, vol. 259, No. 9, p. 5678-5682, 1984; and Zoku-seikagakujikken-koza, 8, blood (I), p. 388-390, Tokyo-Kagaku-Dojin). For example, when an amount of divalent metal ion incorporated in a porphyrin is used as an index, a method using a radioisotope (e.g., ⁵⁹Fe²⁺) as a divalent metal ion, which comprises quantifying an amount of the radioisotope incorporated in a porphyrin, can be employed. In addition, when ferrochelatase produces hem, the amount of hem may be quantified by a pyridine hemochrome method. Furthermore, when zinc ion is used as a divalent metal ion, a method comprising measuring an increase in a specific fluorescence due to zinc porphyrin complex formation can be employed. For example, in the case of zinc protoporphyrin IX, an excitation wavelength of 405 - 420 nm and a fluorescence wavelength of 590 nm can be used. When a production amount of porphyrin complex is used as an index, a decrease in the porphyrin fluorescence due to the porphyrin complex formation can be measured. As the excitation wavelength and the fluorescence wavelength, suitable wavelengths can be selected according to the kind of porphyrin to be used and, in the case of protoporphyrin IX, for example, an excitation wavelength of 405 - 420 nm and a fluorescence wavelength of 620 - 640 nm can be used.

In the above-mentioned screening method, a test substance that increases the ferrochelatase activity by not less than about 20%, preferably not less than about 30%, more preferably not less than about 50%, as compared to the activity in the absence of the test substance, can be selected as a substance that activates ferrochelatase.

The ferrochelatase to be used in the present invention may be a protein derived from a cell (e.g., splenocyte, nerve cell, glial cell, pancreatic β cell, myelocyte, mesangial cell, Langerhans' cell, epidermal cell, epithelial cell, goblet cell, endothelial cell, smooth muscle cell, fibroblast, fibrocyte, myocyte, adipocyte, immunocyte (e.g., macrophage, T cell, B cell, natural killer cell, mast cell, neutrophil, basophil, eosinophil, monocyte, dendritic cell), megakaryocyte, synovial cell, chondrocyte, bone cell, osteoblast, osteoclast, mammary gland cell, hepatocyte, interstitial cell, or a corresponding precursor cell, stem cell or cancer cell thereof) of warm-blooded animals (e.g., human, mouse, rat, guinea pig, hamster, rabbit, sheep, goat, swine, bovine, horse, bird, cat, dog, monkey, chimpanzee) or any tissue in which these cells are present [e.g., brain or any portion of brain (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gallbladder, bone marrow, adrenal gland, skin, muscle, lung, gastrointestinal tract (e.g., large intestine and small intestine), blood vessel, heart, thymus, spleen, submandibular gland, peripheral blood, prostate, testicle, ovary, placenta, uterus, bone, joint, adipose tissue, skeletal muscle, peritoneum]. In addition, the ferrochelatase to be used in the present invention may be also a chemically synthesized protein or a protein synthesized using a cell-free translation system. Alternatively, the ferrochelatase to be used in the present invention may be a recombinant protein produced by a transformant introduced with a nucleic acid having a base sequence that encodes an amino acid sequence of the ferrochelatase.

A specific example of the ferrochelatase to be used in the present invention is a protein comprising an amino acid sequence the same or substantially the same as the amino acid sequence shown by SEQ ID NO: 2 (amino acid sequence of human ferrochelatase shown by GenBank accession number: NM_000140).

As the "substantially the same amino acid sequence" as the amino acid sequence shown by SEQ ID NO: 2, an amino acid sequence having a homology of about 70% or more, preferably about 80% or more, more preferably about 90% or more, particularly preferably about 95% or more, and most preferably about 98% or more, with the amino acid sequence shown by SEQ ID NO: 2 can be mentioned. Here, the "homology" means a ratio (%) of identical amino acid residues and similar amino acid residues to all overlapping amino acid residues in the best alignment where two amino acid sequences are aligned using a mathematical algorithm known in the technical field (preferably, the algorithm considers introduction of gaps on one or both sides of the sequence for the best alignment). "A similar amino acid" means an amino acid having similar physiochemical properties; examples thereof include amino acids classified under the same group, such as aromatic amino acids (Phe, Trp, Tyr), aliphatic amino acids (Ala, Leu, Ile, Val), polar amino acids (Gln, Asn), basic amino acids (Lys, Arg, His), acidic amino acids (Glu, Asp), amino acids having a hydroxyl group (Ser, Thr) and amino acids having a small side-chain (Gly, Ala, Ser, Thr, Met). Substitution by such similar amino acids is expected to give no change in the phenotype of protein (i.e., constitutive amino acid substitution). Specific examples of constitutive amino acid substitution are obvious in the relevant technical field, and are described in various documents (see, for example, Bowie et al., Science, 247:1306-1310 (1990)).

Homology of the amino acid sequences in the present specification can be calculated under the following conditions (an expectation value = 10; gaps are allowed; matrix = BLOSUM62; filtering = OFF) using a homology scoring algorithm NCBI BLAST (National Center for Biotechnology Information Basic Local Alignment Search Tool). As other algorithm for determining the homology of amino acid sequence, for example, the algorithm described in Karlin et al., Proc. Natl. Acad. Sci. USA, 90: 5873-5877 (1993) [the algorithm is incorporated in NBLAST and XBLAST program (version 2.0) (Altschul et al., Nucleic Acids Res., 25: 3389-3402 (1997))], the algorithm described in Needleman et al., J. Mol. Biol., 48: 444-453 (1970) [the algorithm is incorporated in the GAP program in GCG software package], the algorithm described in Myers and Miller, CABIOS, 4: 11-17 (1988) [the algorithm is incorporated in ALIGN program (version 2.0) which is a part of the CGC sequence alignment software package], the algorithm described in Pearson et al., Proc. Natl. Acad. Sci. USA, 85: 2444-2448 (1988) [the algorithm is incorporated in the FASTA program in GCG software package] and the like can be mentioned, and they can be preferably used in a similar way.

More preferably, the "substantially the same amino acid sequence" is an amino acid sequence having an identity of about 70% or more, preferably about 80% or more, more preferably about 90% or more, particularly preferably about 95%, most preferably about 98% or more, with an amino acid sequence shown by SEQ ID NO: 2.

"The protein having substantially the same amino acid sequence" as an amino acid sequence shown by SEQ ID NO: 2 refers a protein that comprises substantially the same amino acid sequence as the aforementioned amino acid sequence shown by SEQ ID NO: 2, and that has substantially the same quality of activity as a protein that comprises an amino acid sequence shown by SEQ ID NO: 2.

The "activity" in the "substantially the same quality of activity" means an activity to produce a porphyrin complex from the aforementioned porphyrin and a divalent metal. The "substantially the same quality" means that the activities are qualitatively the same.

Accordingly, the "protein comprising substantially the same amino acid sequence" preferably has an activity to produce a porphyrin complex, which is equivalent to that of a "protein comprising the amino acid sequence shown by SEQ ID NO: 2". However, the level of the activity may be different (e.g., within the range of about 0.01- to about 100-fold, preferably about 0.1- to about 10-fold, more preferably about 0.5- to about 2-fold, as compared to the activity of the "protein comprising amino acid sequence shown by SEQ ID NO: 2").

In addition, the ferrochelatase to be used in the present invention also includes, for example, a protein comprising
(1) an amino acid sequence shown by SEQ ID NO: 2, except that one or two or more (e.g., about 1-50, preferably about 1-30, more preferably about 1-10, more preferably about 1-5) amino acids have been deleted;
(2) an amino acid sequence shown by SEQ ID NO: 2, except that one or two or more (e.g., about 1-50, preferably about 1-30, more preferably about 1-10, more preferably about 1-5) amino acids have been added;
(3) an amino acid sequence shown by SEQ ID NO: 2, except that one or two or more (e.g., about 1-50, preferably about 1-30, more preferably about 1-10, more preferably about 1-5) amino acids have been inserted;
(4) an amino acid sequence shown by SEQ ID NO: 2, except that one or two or more (e.g., about 1-50, preferably about 1-30, more preferably about 1-10, more preferably about 1-5) amino acids have been substituted by other amino acid(s); or
(5) an amino acid sequence which is a combination thereof; and having substantially the same quality of activity as a protein comprising the amino acid sequence shown by SEQ ID NO: 2. Here, the "substantially the same quality of activity" means as defined above. Accordingly, the above-mentioned deletion, addition, insertion, substitution and a combination thereof of the amino acid need to be qualitatively uninfluential on the activity of ferrochelatase.

For the proteins and peptides described in the present specification, the left end indicates the N-terminus (amino terminus) and the right end indicates the C-terminus (carboxyl terminus), according to the common practice of peptide designation.

For ferrochelatase used for the screening method of the present invention, the C-terminus may be any of a carboxyl group (-COOH), a carboxylate (-COO⁻), an amide (-CONH₂) or an ester (-COOR).

Here, as R in the above-mentioned ester, a C₁₋₆ alkyl group such as methyl, ethyl, n-propyl, isopropyl and n-butyl; a C₃₋₈ cycloalkyl group such as cyclopentyl and cyclohexyl; a C₆₋₁₂ aryl group such as phenyl and α-naphthyl; a phenyl-C₁₋₂ alkyl group such as benzyl and phenethyl; a C₇₋₁₄ aralkyl group such as an α-naphthyl-C₁₋₂ alkyl group such as α-naphthylmethyl; a pivaloyloxymethyl group; and the like can be used.

When the ferrochelatase has a carboxyl group (or a carboxylate) in addition to that on the C-terminal, one in which the carboxyl group is amidated or esterified is also included in the ferrochelatase to be used in the present invention. In this case, as the ester, the above-described C-terminal ester and the like, for example, can be used.

Furthermore, the ferrochelatase to be used in the present invention also includes a protein wherein the amino group of the N-terminal amino acid residue thereof (e.g., methionine residue) is protected by a protecting group (e.g., a C₁₋₆ acyl group such as C₁₋₆ alkanoyl such as a formyl group or an acetyl group, and the like), a protein wherein the N-terminal glutamine residue, which is produced by cleavage in vivo, has been converted to pyroglutamic acid, a protein wherein a substituent (e.g., -OH, -SH, an amino group, an imidazole group, an indole group, a guanidino group and the like) on an amino acid side chain in the molecule is protected by an appropriate protecting group (e.g., a C₁₋₆ acyl group such as a C₁₋₆ alkanoyl group such as a formyl group or an acetyl group, and the like), a conjugated protein such as what is called a glycoprotein, which has a sugar chain bound thereto, and the like.

The ferrochelatase to be used in the present invention may be a partial peptide as long as it has the above-mentioned partial amino acid sequence of ferrochelatase and substantially the same quality of activity as ferrochelatase. Examples of such partial peptide include a peptide comprising the 55th - 423rd amino acid sequence of SEQ ID NO: 2 (i.e., mature region of human ferrochelatase). Here, the "substantially the same quality of activity" means as defined above.

The ferrochelatase to be used in the present invention may be in a free form or a salt. Examples of such salts include physiologically acceptable salts with acids or bases, and physiologically acceptable acid addition salts are particularly preferable. Examples of the acid addition salt include salts with inorganic acids (e.g., hydrochloric acid, phosphoric acid, hydrobromic acid, sulfuric acid), or salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The ferrochelatase can also be prepared from the cells or tissues of the above-described warm-blooded animals by a method of protein purification known per se. Specifically, the ferrochelatase can be prepared by homogenizing a tissue or cells of a warm-blooded animal, and separating and purifying a soluble fraction by chromatographies such as reversed-phase chromatography, ion exchange chromatography, affinity chromatography and the like.

The ferrochelatase can also be produced according to known peptide synthesis method.

The method of peptide synthesis may be any of, for example, a solid phase synthesis process and a liquid phase synthesis process. That is, a desired protein can be produced by condensing a partial peptide or amino acids capable of constituting the ferrochelatase and the remaining portion, and eliminating any protecting group the resultant product may have.

As examples of the commonly known methods of condensation and elimination of the protecting group, the methods described in (1) to (5) below can be mentioned.
(1) M. Bodanszky and M.A. Ondetti, Peptide Synthesis, Interscience Publishers, New York (1966)
(2) Schroeder and Luebke, The Peptide, Academic Press, New York (1965)
(3) Nobuo Izumiya, et al.: Peptide Gosei-no-Kiso to Jikken, published by Maruzen Co. (1975);
(4) Haruaki Yajima and Shunpei Sakakibara: Seikagaku Jikken Koza 1, Tanpakushitsu no Kagaku IV, 205 (1977)
(5) Haruaki Yajima, ed.: Zoku Iyakuhin no Kaihatsu, Vol. 14, Peptide Synthesis, published by Hirokawa Shoten.

The ferrochelatase thus obtained can be isolated and purified by a known purification method.

Moreover, a ferrochelatase can also be produced by culturing a transformant having the nucleic acid encoding same, and separating and purifying a ferrochelatase from the obtained culture.

The nucleic acid that encodes ferrochelatase may be DNA or RNA, or a DNA/RNA chimera, and is preferably DNA. In addition, the nucleic acid may be a double-strand, or single-strand. The double-strand may be a double-stranded DNA, a double-stranded RNA, or a DNA:RNA hybrid. In the case of a single strand, it may be a sense strand (i.e., coding strand) or an antisense strand (i.e., non-coding strand).

As the DNA encoding a ferrochelatase, genomic DNA, cDNA derived from any cell [for example, splenocyte, nerve cell, glial cell, pancreatic β cells, myeloid cell, mesangial cell, Langerhans' cell, epidermal cell, epithelial cell, endothelial cell, fibroblast, fibrocyte, myocytes, adipocyte, immunocyte (e.g., macrophage, T cell, B cell, natural killer cell, mast cell, neutrophil, basophil, eosinophils, monocyte, dendritic cell), megakaryocyte, synovial cell, chondrocytes, bone cell, osteoblast, osteoclast, mammary cell, hepatocyte or interstitial cell, or a corresponding precursor cell, stem cell, cancer cell and the like, blood lineage cells] of warm-blooded animal (e.g., human, mouse, rat, guinea pig, hamster, rabbit, sheep, goat, swine, bovine, horse, bird, cat, dog, monkey, chimpanzee), or any tissue where such cells are present [e.g., brain or any portion of brain (e.g., olfactory bulb, amygdaloid nucleus, basal ganglia, hippocampus, thalamus, hypothalamus, subthalamic nucleus, cerebral cortex, medulla oblongata, cerebellum, occipital lobe, frontal lobe, lateral lobe, putamen, caudate nucleus, callosum, substantia nigra), spinal cord, hypophysis, stomach, pancreas, kidney, liver, gonad, thyroid, gallbladder, bone marrow, adrenal gland, skin, muscle, lung, gastrointestinal tract (e.g., large intestine, small intestine), blood vessel, heart, thymus, spleen, submandibular gland, peripheral blood, peripheral blood cell, prostate, testicle, ovary, placenta, uterus, bone, joint, adipose tissue, peritoneum], synthetic DNA and the like can be mentioned.

A genomic DNA and cDNA encoding ferrochelatase can also be directly amplified according to a method known *per se*, for example, Polymerase Chain Reaction (hereinafter to be abbreviated as "PCR method") or Reverse Transcriptase-PCR (hereinafter to be abbreviated as "RT-PCR method"), using genomic DNA fraction or total RNA or mRNA fraction prepared from the above-mentioned cell/tissue as a template. Alternatively, genomic DNA or cDNA encoding ferrochelatase can also be cloned according to a method known *per se*, for example, colony or plaque hybridization method, PCR method and the like, from the genomic DNA library or cDNA library prepared by inserting, into a suitable vector, a fragment of genomic DNA or total RNA or mRNA prepared from the above-mentioned cell/tissue. The vector to be used for the library may be any of bacteriophage, plasmid, cosmid, phagemid and the like.

As the DNA encoding a ferrochelatase, a DNA having the base sequence shown by SEQ ID NO: 1 (base sequence of nucleic acid encoding the amino acid sequence of human ferrochelatase shown by SEQ ID NO: 2), a DNA encoding a protein having a base sequence hybridizing to a complementary strand sequence of the base sequence shown by SEQ ID NO: 1 under high stringent conditions and having substantially the same quality of activity as the aforementioned protein comprising the amino acid sequence shown by SEQ ID NO: 2 (i.e., an activity to produce protoheme from protoporphyrin IX and a divalent iron), and the like can be mentioned.

Useful DNA capable of hybridizing with the complementary strand sequence of the base sequence shown by SEQ ID NO:1 under high stringent conditions include, for example, a DNA comprising a base sequence having a homology of about 60% or more, preferably about 70% or more, more preferably about 80% or more, particularly preferably about 90% or more, to the base sequence shown by SEQ ID NO: 1.

Homology of the base sequences in the present specification can be calculated under the following conditions (an expectation value = 10; gaps are allowed; filtering = ON; match score = 1; mismatch score = -3) using a homology scoring algorithm NCBI BLAST (National Center for Biotechnology Information Basic Local Alignment Search Tool). As other algorithm with which to determine the homology of the base sequence, the homology calculation algorithm of the above-mentioned amino acid sequence can be preferably used in the same manner.

The hybridization can be performed by a method known per se or a method analogous thereto, for example, a method described in Molecular Cloning, 2nd ed. (J. Sambrook et al., Cold Spring Harbor Lab. Press, 1989) and the like. A commercially available library can also be used for the hybridization according to the instructions of the attached manufacturer's protocol. Preferably, the hybridization can be carried out under high stringent conditions.

High-stringent conditions refer to, for example, conditions involving a sodium salt concentration of about 19 to about 40 mM, preferably about 19 to about 20 mM, and a temperature of about 50 to about 70°C, preferably about 60 to about 65°C. In particular, a case wherein the sodium concentration is about 19mM and the temperature is about 65°C is preferred. Those skilled in the art can easily regulate the conditions to obtain a desired stringency by appropriately changing the salt concentration of hybridization solution, hybridization reaction temperature, probe concentration, probe length, number of mismatches, hybridization reaction time, salt concentration of washing solution, washing temperature, and the like.

The DNA encoding ferrochelatase is preferably human ferrochelatase DNA having the base sequence shown by SEQ ID NO: 1, or its allele variant, or ortholog of other warm-blooded animal (e.g., mouse, rat, guinea pig, hamster, rabbit, sheep, goat, swine, bovine, horse, bird, cat, dog, monkey, chimpanzee).

The DNA that encodes the ferrochelatase or a partial peptide thereof can be cloned by amplifying it by the PCR method using a synthetic DNA primer having a portion of the base sequence that encodes the protein or peptide, or by hybridizing DNA incorporated in an appropriate expression vector to a labeled DNA fragment or synthetic DNA that encodes a portion or the entire region of the ferrochelatase protein. The hybridization can be performed by, for example, a method described in Molecular Cloning, 2nd ed. (ibid.) and the like. A commercially available library can also be used for the hybridization according to the instructions of the manufacturer's protocol attached thereto.

The base sequence of the DNA can be converted according to a method known per se, such as the ODA-LA PCR method, the Gapped duplex method, or the Kunkel method, or a method based thereon, using a commonly known kit, for example, Mutan^{™}-super Express Km (TAKARA SHUZO CO. LTD.), Mutan^{™}-K (TAKARA SHUZO CO. LTD.) and the like.

The cloned DNA can be used as is, or after digestion with a restriction endonuclease or addition of a linker as desired, depending on the purpose of its use. The DNA may have the translation initiation codon ATG at the 5' end thereof, and the translation stop codon TAA, TGA or TAG at the 3' end thereof. These translation initiation codons and translation stop codons can be added by using a suitable synthetic DNA adaptor.

The ferrochelatase can also be prepared by transforming a host with an expression vector containing the above-mentioned DNA encoding ferrochelatase, and cultivating the obtained transformant, according to a method known per se.

Examples of the test substance to be used for the screening method of the present invention include peptide, protein, nonpeptidic compound, fermentation product, cell extract, plant extract, animal tissue extract and the like, and these may be novel substances or known substances.

Since the substance obtained by the screening method of the present invention can promote an activity to produce a porphyrin complex from a porphyrin and a divalent metal (e.g., activity to produce protoheme from protoporphyrin IX and a divalent iron), it can be used as a ferrochelatase activator. In the following, "the substance obtained by the screening method of the present invention" and "a ferrochelatase activator containing compound (I)" are sometimes to be abbreviated as the ferrochelatase activator of the present invention as a generic term.

Ferrochelatase produces hem in mitochondria, and supplies hem to various proteins (e.g., proteins involved in the electron transport chain such as cytochrome c oxidase and the like, catalase involved in radical treatment). Therefore, a ferrochelatase activator improves mitochondrial function by activating the proteins involved in the electron transport chain by promotion of hem supply to the proteins and the like, while promoting the hem supply to catalase, whereby the intracellular redox control activity can be improved. Here, examples of the mitochondrial function include substrate channel function, glycolipid metabolism, energy production (e.g., ATP production), regulation of apoptosis, Ca-buffering function and the like.

The ferrochelatase activator of the present invention can improve the neuronal function impaired by the pathology when its action reaches a nerve tissue, and can also improve the glycolipid metabolism when its action reaches a peripheral tissue such as liver and the like. Thus, the ferrochelatase activator of the present invention is effective for the prophylaxis or treatment of a disease showing a decreased mitochondrial function in the tissue. Here, examples of the tissue include the brain and each part of the brain (e.g., olfactory bulb, amygdaloid nucleus, basal ganglia, hippocampus, thalamus, hypothalamus, cerebral cortex, medulla oblongata, cerebellum), spinal cord, pituitary gland, stomach, pancreas, kidney, liver, gonad, thyroid gland, gallbladder, bone marrow, adrenal gland, skin, muscle, lung, gastrointestinal tract (e.g., large intestine, small intestine), blood vessel, heart, thymus, spleen, submandibular gland, peripheral blood, prostate, testis, ovary, placenta, uterus, bone, articular, skeletal muscle and the like. In addition, the decreased mitochondrial function is a state showing not more than about 80%, preferably not more than about 70%, more preferably not more than about 60%, of the above-mentioned mitochondrial function as compared to a healthy control.

Specifically, the ferrochelatase activator of the present invention can be used for the prophylaxis or treatment of a disease showing a decreased mitochondrial function in a tissue (e.g., diabetes, nerve system disease, mitochondrial encephalomyopathy, fibromyalgia, chronic fatigue syndrome). Here, the diabetes includes type 1 diabetes, type 2 diabetes, gestational diabetes and the like. In addition, the nerve system disease means abnormality, disorder or injury of the brain, spinal cord, autonomic ganglion, central nervous system, peripheral part, cranial nerve, spinal nerve, plexus and the like. Specific examples thereof include amyotropic lateral sclerosis retinitis, pigmentosa, cerebellar degeneration, diabetic neuropathy, neuropathic pain, multiple sclerosis, Alzheimer-type senile dementia, Parkinson's disease, Huntington chorea, schizophrenia, Down's syndrome and the like. In addition, specific examples of the nerve system disease also include neuropathy and the like due to cancer therapy.

In addition, the ferrochelatase activator of the present invention can also be used for the prophylaxis or treatment of a disorder of an organ showing a decreased mitochondrial function of a tissue (e.g., pancreatic exhaustion, steatohepatitis).

Furthermore, since ferrochelatase supplies hem to hemoglobin, the ferrochelatase activator of the present invention is useful for the prophylaxis or treatment of a disease caused by a decrease in the hem in the body (e.g., anemia).

Specific examples of the substance obtained by the screening method of the present invention include a compound represented by the formula: 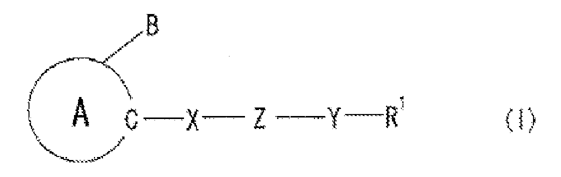 wherein ring A is a 5-membered aromatic heterocycle containing two or more nitrogen atoms and optionally further having substituent(s);
B is an optionally substituted hydrocarbon group or an optionally substituted heterocyclic group;
X is a divalent noncyclic hydrocarbon group;
Z is -O-, -S-, -NR²-, -CONR²- or -NR²CO- wherein R² is a hydrogen atom or an optionally substituted alkyl group;
Y is a bond or a divalent noncyclic hydrocarbon group; and
R¹ is an optionally substituted cyclic group, an optionally substituted amino group or an optionally substituted acyl group, or a salt thereof.

In the formula (I), examples of the "5-membered aromatic heterocycle containing two or more nitrogen atoms" of the "5-membered aromatic heterocycle containing two or more nitrogen atoms and optionally further having substituent(s)" for ring A include a 5-membered aromatic heterocycle containing, as a ring constituting atoms, two or more nitrogen atoms besides carbon atom, and optionally further containing 1 or 2 hetero atoms selected from an oxygen atom, a sulfur atom and a nitrogen atom.

Specific examples of the "5-membered aromatic heterocycle containing two or more nitrogen atoms" include imidazole, pyrazole, oxadiazole, thiadiazole, triazole, tetrazole rings and the like. Of these, pyrazole, oxadiazole, thiadiazole, triazole and tetrazole rings are preferable, and a pyrazole ring is particularly preferable.

The "5-membered aromatic heterocycle containing two or more nitrogen atoms" optionally further contains 1 or 2 substituents at substitutable position(s). Examples of such substituent include a halogen atom, an optionally substituted hydrocarbon group, an optionally substituted heterocyclic group, an optionally substituted hydroxy group, an optionally substituted mercapto group, an optionally substituted amino group and the like.

In the present specification, examples of the "halogen atom" include fluorine, chlorine, bromine and iodine atoms.

Examples of the "hydrocarbon group" in the aforementioned "optionally substituted hydrocarbon group" include an aliphatic hydrocarbon group, an alicyclic hydrocarbon group, an aromatic hydrocarbon group, an aromatic aliphatic hydrocarbon group, an alicyclic aliphatic hydrocarbon group and the like.

Examples of the aliphatic hydrocarbon group include a linear or branched C₁₋₁₅ aliphatic hydrocarbon group, specifically, an alkyl group, an alkenyl group, an alkynyl group and the like.

Preferable examples of the alkyl group include a C₁₋₁₀ alkyl group such as methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, heptyl, octyl, nonyl, decyl and the like.

Preferable examples of the alkenyl group include a C₂₋₁₀ alkenyl group such as ethenyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl, 5-hexenyl, 1-heptenyl, 1-octenyl and the like.

Preferable examples of the alkynyl group include a C₂₋₁₀ alkynyl group such as ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, 1-heptynyl, 1-octynyl and the like.

Examples of the alicyclic hydrocarbon group include a saturated or unsaturated C₃₋₁₂ alicyclic hydrocarbon group, specifically, a cycloalkyl group, a cycloalkenyl group, a cycloalkadienyl group and the like.

Preferable examples of the cycloalkyl group include a C₃₋₁₀ cycloalkyl group such as cyclopropyl, cyclobutyl, cyclopentyl, cyclohexyl, cycloheptyl, cyclooctyl, bicyclo[2.2.1]heptyl, bicyclo[2.2.2]octyl, bicyclo[3.2.1]octyl, bicyclo[3.2.2]nonyl, bicyclo[3.3.1]nonyl, bicyclo[4.2.1]nonyl, bicyclo[4.3.1]decyl and the like.

Preferable examples of the cycloalkenyl group include a C₃₋₁₀ cycloalkenyl group such as 2-cyclopenten-1-yl, 3-cyclopenten-1-yl, 2-cyclohexen-1-yl, 3-cyclohexen-1-yl and the like.

Preferable examples of the cycloalkadienyl group include a C₄₋₁₀ cycloalkadienyl group such as 2,4-cyclopentadien-1-yl, 2,4-cyclohexadien-1-yl, 2,5-cyclohexadien-1-yl and the like.

Examples of the aromatic hydrocarbon group include a C₆₋₁₄ aryl group. Preferable examples of the aryl group include phenyl, naphthyl, anthryl, phenanthryl, acenaphthyl, biphenylyl, indenyl and the like. Of these, phenyl, naphthyl and the like are preferable. The aryl group may be partially saturated, and examples of the partially saturated aryl group include dihydroindenyl and the like.

Examples of the aromatic aliphatic hydrocarbon group include a C₇₋₁₃ aromatic aliphatic hydrocarbon group, specifically, an aralkyl group, an arylalkenyl group and the like.

Preferable examples of the aralkyl group include a C₇₋₁₃ aralkyl group such as benzyl, phenethyl, phenylpropyl, naphthylmethyl, benzhydryl and the like.

Preferable examples of the arylalkenyl group include a C₈₋₁₃ arylalkenyl group such as styryl and the like.

Examples of the alicyclic aliphatic hydrocarbon group include a C₄₋₁₃ alicyclic aliphatic hydrocarbon group, specifically a cycloalkylalkyl group, a cycloalkylalkenyl group and the like.

Preferable examples of the cycloalkylalkyl group include a C₄₋₁₃ cycloalkylalkyl group such as cyclopropylmethyl, cyclopropylethyl, cyclopentylmethyl, cyclopentylethyl, cyclohexylmethyl, cyclohexylethyl and the like.

Preferable examples of the cycloalkylalkenyl group include a C₅₋₁₃ cycloalkylalkenyl group such as cyclopropylethenyl, cyclopentylethenyl, cyclohexylethenyl and the like.

The above-mentioned "hydrocarbon group" optionally has 1 to 3 substituents at substitutable position(s). Examples of such substituent include a halogen atom, nitro, oxo, C₁₋₃ alkylenedioxy, an optionally substituted aromatic heterocyclic group, an optionally substituted nonaromatic heterocyclic group, an optionally substituted amino group, an optionally substituted hydroxy group, an optionally substituted mercapto group, an optionally substituted acyl group and the like.

Examples of the C₁₋₃ alkylenedioxy include methylenedioxy, ethylenedioxy and the like.

Examples of the "aromatic heterocyclic group" of the "optionally substituted aromatic heterocyclic group" include a 5- to 7-membered monocyclic aromatic heterocyclic group containing, as ring constituting atoms besides carbon atom, 1 to 4 hetero atoms selected from an oxygen atom, a sulfur atom and a nitrogen atom and a fused aromatic heterocyclic group. Examples of the fused aromatic heterocyclic group include a group obtained by condensation of such 5- to 7-membered monocyclic aromatic heterocyclic group with a 6-membered ring containing 1 or 2 nitrogen atoms, a benzene ring or a 5-membered ring containing one sulfur atom, and the like.

Preferable examples of the "aromatic heterocyclic group" include furyl (e.g., 2-furyl, 3-furyl), thienyl (e.g., 2-thienyl, 3-thienyl), pyridyl (e.g., 2-pyridyl, 3-pyridyl, 4-pyridyl), pyrimidinyl (e.g., 2-pyrimidinyl, 4-pyrimidinyl, 5-pyrimidinyl, 6-pyrimidinyl), pyridazinyl (e.g., 3-pyridazinyl, 4-pyridazinyl), pyrazinyl (e.g., 2-pyrazinyl), pyrrolyl (e.g., 1-pyrrolyl, 2-pyrrolyl, 3-pyrrolyl), imidazolyl (e.g., 1-imidazolyl, 2-imidazolyl, 4-imidazolyl, 5-imidazolyl), pyrazolyl (e.g., 1-pyrazolyl, 3-pyrazolyl, 4-pyrazolyl), oxazolyl (e.g., 2-oxazolyl, 4-oxazolyl, 5-oxazolyl), isoxazolyl, thiazolyl (e.g., 2-thiazolyl, 4-thiazolyl, 5-thiazolyl), isothiazolyl, oxadiazolyl (e.g., 1,2,4-oxadiazol-5-yl, 1,3,4-oxadiazol-2-yl), thiadiazolyl (e.g., 1,3,4-thiadiazol-2-yl), triazolyl (e.g., 1,2,4-triazol-1-yl, 1,2,4-triazol-3-yl, 1,2,4-triazol-5-yl, 1,2,3-triazol-1-yl, 1,2,3-triazol-2-yl, 1,2,3-triazol-4-yl), tetrazolyl (e.g., tetrazol-1-yl, tetrazol-5-yl), quinolyl (e.g., 2-quinolyl, 3-quinolyl, 4-quinolyl), quinazolyl (e.g., 2-quinazolyl, 4-quinazolyl), quinoxalyl (e.g., 2-quinoxalyl), benzofuryl (e.g., 2-benzofuryl, 3-benzofuryl), benzothienyl (e.g., 2-benzothienyl, 3-benzothienyl), benzoxazolyl (e.g., 2-benzoxazolyl), benzothiazolyl (e.g., 2-benzothiazolyl), benzimidazolyl (e.g., benzimidazol-1-yl, benzimidazol-2-yl), indolyl (e.g., indol-1-yl, indol-3-yl), 1H-indazolyl (e.g., 1H-indazol-3-yl), 1H-pyrrolo[2,3-b]pyrazinyl (e.g., 1H-pyrrolo[2,3-b]pyrazin-2-yl), 1H-pyrrolopyridinyl (e.g., 1H-pyrrolo[2,3-b]pyridin-6-yl), 1H-imidazopyridinyl (e.g., 1H-imidazo[4,5-b]pyridin-2-yl, 1H-imidazo[4,5-c]pyridin-2-yl), 1H-imidazopyrazinyl (e.g., 1H-imidazo[4,5-b]pyrazin-2-yl), triazinyl, isoquinolyl, benzooxadiazolyl, benzothiadiazolyl, benztriazolyl and the like.

Examples of the "nonaromatic heterocyclic group" of the "optionally substituted nonaromatic heterocyclic group" include a 5- to 7-membered monocyclic nonaromatic heterocyclic group containing, as ring constituting atoms besides carbon atom, 1 to 4 hetero atoms selected from an oxygen atom, a sulfur atom and a nitrogen atom, and a fused nonaromatic heterocyclic group. Examples of the fused nonaromatic heterocyclic group include a group obtained by condensation of such 5- to 7-membered monocyclic nonaromatic heterocyclic group with a 6-membered ring containing 1 or 2 nitrogen atoms, a benzene ring or a 5-membered ring containing one sulfur atom and the like.

Preferable examples of the "nonaromatic heterocyclic group" include pyrrolidinyl (e.g., 1-pyrrolidinyl), piperidinyl (e.g., piperidino), morpholinyl (e.g., morpholino), thiomorpholinyl (e.g., thiomorpholino), piperazinyl (e.g., 1-piperazinyl), hexamethyleniminyl (e.g., hexamethyleneimine-1-yl), oxazolidinyl (e.g., oxazolidin-3-yl), thiazolidinyl (e.g., thiazolidin-3-yl), imidazolidinyl (e.g., imidazolidin-3-yl), imidazolinyl (e.g., imidazolin-1-yl, imidazolin-2-yl), oxazolinyl (e.g., oxazolin-2-yl), thiazolinyl (e.g., thiazolin-2-yl), oxazinyl (e.g., oxazin-2-yl), tetrahydrofuranyl, azepanyl, tetrahydropyridinyl (e.g., 1,2,3,6-tetrahydropyridin-1-yl), dihydrobenzofuranyl, dioxolanyl, dithioranyl, dioxothiazolidinyl, dioxooxazolidinyl and the like.

The above-mentioned aromatic heterocyclic group and nonaromatic heterocyclic group optionally have 1 to 3 substituents at substitutable position(s). Examples of such substituent include nitro, hydroxy, amino, oxo, a halogen atom, C₁₋₆ alkyl optionally substituted by 1 to 3 halogen atoms, a C₁₋₆ alkoxy group optionally substituted by 1 to 3 halogen atoms, C₆₋₁₄ aryl (e.g., phenyl) and the like.

Examples of the "optionally substituted amino group" include an amino group optionally mono- or di-substituted by a C₁₋₁₀ alkyl group, a C₂₋₁₀ alkenyl group, a C₃₋₁₀ cycloalkyl group, a C₃₋₁₀ cycloalkenyl group, a C₆₋₁₄ aryl group, a C₇₋₁₃ aralkyl group, a C₁₋₁₃ acyl group or a heteroaryl group, each optionally having substituent(s).

Here, examples of the C₁₋₁₀ alkyl group, C₂₋₁₀ alkenyl group, C₃₋₁₀ cycloalkyl group, C₃₋₁₀ cycloalkenyl group, C₆₋₁₄ aryl group and C₇₋₁₃ aralkyl group include those exemplified as the "hydrocarbon group" of the "optionally substituted hydrocarbon group" exemplified as the substituents for the ring A.

Examples of the aforementioned C₁₋₁₃ acyl group include those exemplified as the acyl group of the below-mentioned "optionally substituted acyl group". The acyl group is preferably formyl, C₁₋₁₀ alkyl-carbonyl, C₁₋₆ alkoxy-carbonyl, C₆₋₁₄ aryl-carbonyl, C₇₋₁₃ aralkyl-carbonyl, 5- or 6-membered aromatic heterocyclyl-carbonyl, 5- or 6-membered non-aromatic heterocyclyl-carbonyl and the like.

Here, preferable examples of the C₁₋₁₀ alkyl-carbonyl include acetyl, propionyl, butyryl, isobutyryl, valeryl, isovaleryl, pivaloyl, hexanoyl, heptanoyl, octanoyl and the like.

Preferable examples of the C₁₋₆ alkoxy-carbonyl include tert-butoxycarbonyl and the like.

Preferable examples of the C₆₋₁₄ aryl-carbonyl include benzoyl and the like.

Preferable examples of the C₇₋₁₃ aralkyl-carbonyl include benzylcarbonyl, phenethylcarbonyl and the like.

Preferable examples of the 5- or 6-membered aromatic heterocyclyl-carbonyl include furylcarbonyl, pyrrolylcarbonyl, thienylcarbonyl, pyridylcarbonyl and the like.

Preferable examples of the 5- or 6-membered non-aromatic heterocyclyl-carbonyl include tetrahydrofurylcarbonyl and the like.

Examples of the aforementioned heteroaryl group include aromatic heterocyclic group exemplified as the substituents of the "optionally substituted hydrocarbon group" exemplified as the substituents of ring A. Of these, pyridyl, imidazolyl, triazolyl, pyrimidinyl and the like are preferable.

These C₁₋₁₀ alkyl group, C₂₋₁₀ alkenyl group, C₃₋₁₀ cycloalkyl group, C₃₋₁₀ cycloalkenyl group, C₆₋₁₄ aryl group, C₇₋₁₃ aralkyl group, C₁₋₁₃ acyl group and heteroaryl group may have 1 to 6, preferably 1 or 2, substituents at substitutable position(s). Examples of the substituent include a halogen atom, a C₁₋₆ alkyl group optionally substituted by 1 to 3 halogen atoms, a C₁₋₆ alkoxy group optionally substituted by 1 to 3 halogen atoms, hydroxy, nitro, amino, a C₁₋₆ alkylsulfonyl group and the like.

As for the "optionally substituted hydroxy group", examples of the "substituted hydroxy group" include an alkoxy group, an alkenyloxy group, a cycloalkyloxy group, a cycloalkenyloxy group, an aryloxy group, an aralkyloxy group, an acyloxy group, a heteroaryloxy group and the like, which are each optionally substituted.

Preferable examples of the alkoxy group include a C₁₋₁₀ alkoxy group such as methoxy, ethoxy, propoxy, isopropoxy, butoxy, isobutoxy, sec-butoxy, tert-butoxy, pentyloxy, isopentyloxy, neopentyloxy, hexyloxy, heptyloxy, nonyloxy and the like.

Preferable examples of the alkenyloxy group include a C₂₋₁₀ alkenyloxy group such as allyloxy, crotyloxy, 2-pentenyloxy, 3-hexenyloxy and the like.

Preferable examples of the cycloalkyloxy group include a C₃₋₁₀ cycloalkyloxy group such as cyclobutoxy, cyclopentyloxy, cyclohexyloxy and the like.

Preferable examples of the cycloalkenyloxy group include a C₃₋₁₀ cycloalkenyloxy group such as 2-cyclopentenyloxy, 2-cyclohexenyloxy and the like.

Preferable examples of the aryloxy group include a C₆₋₁₄ aryloxy group such as phenoxy, naphthyloxy and the like.

Preferable examples of the aralkyloxy group include a C₇₋₁₃ aralkyloxy group such as benzyloxy, phenethyloxy, naphthylmethyloxy and the like.

Preferable examples of the acyloxy group include a C₂₋₁₃ acyloxy group such as a C₁₋₆ alkyl-carbonyloxy group (e.g., acetyloxy, propionyloxy, butyryloxy, isobutyryloxy) and the like.

Preferable examples of the heteroaryloxy group include a 5- to 7-membered monocyclic heteroaryloxy group such as 2-pyridyloxy, 3-pyridyloxy, 2-imidazolyloxy, 2-pyrimidinyloxy, 1,2,4-triazol-5-yloxy and the like.

The above-mentioned alkoxy group, alkenyloxy group, cycloalkyloxy group, cycloalkenyloxy group, aryloxy group, aralkyloxy group, acyloxy group and heteroaryloxy group may have 1 to 3, preferably 1 or 2, substituents at substitutable position(s). Examples of the substituent include a halogen atom, a C₁₋₆ alkyl group optionally substituted by 1 to 3 halogen atoms, a C₁₋₆ alkoxy group optionally substituted by 1 to 3 halogen atoms, hydroxy, nitro, amino, a C₁₋₆ alkylsulfonyl group and the like can be mentioned.

As for the "optionally substituted mercapto group", examples of the "substituted mercapto group" include an alkylthio group, an alkenylthio group, a cycloalkylthio group, a cycloalkenylthio group, an arylthio group, an aralkylthio group, an acylthio group, a heteroarylthio group and the like, which are each optionally substituted.

Preferable examples of the alkylthio group include a C₁₋₁₀ alkylthio group such as methylthio, ethylthio, propylthio, isopropylthio, butylthio, isobutylthio, sec-butylthio, tert-butylthio, pentylthio, isopentylthio, neopentylthio, hexylthio, heptylthio, nonylthio and the like.

Preferable examples of the alkenylthio group include a C₂₋₁₀ alkenylthio group such as allylthio, crotylthio, 2-pentenylthio, 3-hexenylthio and the like.

Preferable examples of the cycloalkylthio group include a C₃₋₁₀ cycloalkylthio group such as cyclobutylthio, cyclopentylthio, cyclohexylthio and the like.

Preferable examples of the cycloalkenylthio group include a C₃₋₁₀ cycloalkenylthio group such as 2-cyclopentenylthio, 2-cyclohexenylthio and the like.

Preferable examples of the arylthio group include a C₆₋₁₄ arylthio group such as phenylthio, naphthylthio and the like.

Preferable examples of the aralkylthio group include a C₇₋₁₃ aralkylthio group such as benzylthio, phenethylthio, naphthylmethylthio and the like.

Preferable examples of the acylthio group include a C₂₋₁₃ acylthio group such as a C₁₋₆ alkyl-carbonylthio group (e.g., acetylthio, propionylthio, butyrylthio, isobutyrylthio) and the like.

Preferable examples of the heteroarylthio group include a 5- to 7-membered monocyclic heteroarylthio group such as 2-pyridylthio, 3-pyridylthio, 2-imidazolylthio, 2-pyrimidinylthio, 1,2,4-triazol-5-ylthio and the like.

The above-mentioned alkylthio group, alkenylthio group, cycloalkylthio group, cycloalkenylthio group, arylthio group, aralkylthio group, acylthio group and heteroarylthio group may have 1 or 2 substituents at substitutable position(s). Examples of the substituent include halogen atom, a C₁₋₆ alkyl group optionally substituted by 1 to 3 halogen atoms, a C₁₋₆ alkoxy group optionally substituted by 1 to 3 halogen atoms, hydroxy, nitro, amino, a C₁₋₆ alkylsulfonyl group, oxo and the like.

The acyl group of the "optionally substituted acyl group" is, for example, a group represented by the formula: -COR⁴, - CO-OR⁴, -SO₂R⁴, -SOR⁴, -PO₃R⁴R⁵ [i.e., -P(=O)(OR⁴)(OR⁵)], -CO-NR^{4a}R^{5a}, -CS-NR^{4a}R^{5a}, -SO₂-NR^{4a}R^{5a} wherein R⁴ and R⁵ are the same or different and each is a hydrogen atom, a hydrocarbon group or a heterocyclic group, R⁴ and R⁵ optionally form a heterocycle together with the adjacent oxo-substituted phosphorus atom and two oxygen atoms, R^{4a} and R^{5a} are the same or different and each is a hydrogen atom, a hydrocarbon group or a heterocyclic group, or R^{4a} and R^{5a} optionally form a nitrogen-containing heterocycle together with the adjacent nitrogen atom, and the like.

Examples of the "hydrocarbon group" for R⁴, R⁵, R^{4a} or R^{5a} include those exemplified as the "hydrocarbon group" of the "optionally substituted hydrocarbon group" exemplified as the substituents of ring A.

The hydrocarbon group is preferably a C₁₋₁₀ alkyl group; a C₂₋₁₀ alkynyl group; a C₃₋₁₀ cycloalkyl group optionally fused with a benzene ring; a C₆₋₁₄ aryl group optionally fused with C₃₋₁₀ cycloalkane (preferably phenyl, dihydroindenyl, biphenylyl); a C₇₋₁₃ aralkyl group and the like.

Examples of the "heterocyclic group" for R⁴, R⁵, R^{4a} or R^{5a} include aromatic heterocyclic group and nonaromatic heterocyclic group exemplified as the substituents of the "optionally substituted hydrocarbon group" exemplified as the substituents of ring A.

The heterocyclic group is preferably thiazolyl, oxazolyl, isothiazolyl, isoxazolyl, pyrazolyl, pyridyl, pyrazinyl, benzoxazolyl, benzothiazolyl, benzothiadiazolyl, quinolyl, isoquinolyl, pyrrolidinyl, piperidinyl, piperazinyl and the like.

The heterocycle formed by R⁴ and R⁵ together with the adjacent oxo-substituted phosphorus atom and two oxygen atoms is, for example, a 4- to 7-membered heterocycle containing, as a ring constituting atom besides carbon atom, an oxo-substituted phosphorus atom and two oxygen atoms, and optionally further containing 1 or 2 hetero atoms selected from an oxygen atom, a nitrogen atom and a sulfur atom and the like. Specific examples of the heterocycle include 2-oxide-1,3,2-dioxaphosphinane, 2-oxide-1,3,2-dioxaphosphorane, 2-oxide-4,7-dihydro-1,3,2-dioxaphosphepine and the like.

The "nitrogen-containing heterocycle" formed by R^{4a} and R^{5a} together with the adjacent nitrogen atom is, for example, a 5- to 7-membered nitrogen-containing heterocycle containing, as a ring constituting atom besides carbon atom, at least one nitrogen atom, and optionally further containing 1 or 2 hetero atoms selected from an oxygen atom, a sulfur atom and a nitrogen atom. Preferable examples of the nitrogen-containing heterocycle include pyrrolidine, imidazolidine, pyrazolidine, piperidine, piperazine, morpholine, thiomorpholine and the like.

The acyl group may have 1 to 3 substituents at substitutable position(s). Examples of the substituent include C₁₋₆ alkyl optionally substituted by 1 to 3 halogen atoms, C₁₋₆ alkoxy optionally substituted by 1 to 3 halogen atoms, a halogen atom, nitro, hydroxy, amino optionally mono- or di-substituted by C₁₋₆ alkyl, and the like.

Preferable examples of the acyl group include formyl, carboxyl, carbamoyl, thiocarbamoyl, C₁₋₁₀ alkyl-carbonyl (e.g., acetyl, propionyl, butyryl, isobutyryl, valeryl, isovaleryl, pivaloyl, hexanoyl, heptanoyl, octanoyl), C₂₋₁₀ alkenyl-carbonyl (e.g., crotonoyl), C₃₋₁₀ cycloalkyl-carbonyl (e.g., cyclobutanecarbonyl, cyclopentanecarbonyl, cyclohexanecarbonyl, cycloheptanecarbonyl), C₃₋₁₀ cycloalkenyl-carbonyl (e.g., 2-cyclohexenecarbonyl), C₆₋₁₄ aryl-carbonyl (e.g., benzoyl, 1-naphthoyl, 2-naphthoyl), C₇₋₁₃ aralkyl-carbonyl (e.g., benzylcarbonyl, phenethylcarbonyl), aromatic heterocyclylcarbonyl (e.g., nicotinoyl, isonicotinoyl), non-aromatic heterocyclylcarbonyl (e.g., pyrrolidinylcarbonyl, piperidinocarbonyl), C₁₋₆ alkoxy-carbonyl (e.g., methoxycarbonyl, ethoxycarbonyl, propoxycarbonyl, butoxycarbonyl, tert-butoxycarbonyl), C₆₋₁₄ aryloxy-carbonyl (e.g., phenyloxycarbonyl, naphthyloxycarbonyl), C₇₋₁₃ aralkyloxy-carbonyl (e.g., benzyloxycarbonyl, phenethyloxycarbonyl), mono- or di-(C₁₋₆ alkyl optionally having 1 to 3 substituents selected from a halogen atom and C₁₋₆ alkoxy-carbonyl)-carbamoyl (e.g., methylcarbamoyl, ethylcarbamoyl, dimethylcarbamoyl, diethylcarbamoyl, ethylmethylcarbamoyl, propylcarbamoyl, trifluoroethylcarbamoyl), mono- or di-(C₁₋₆ alkyl optionally substituted by 1 to 3 halogens)-thiocarbamoyl (e.g., methylthiocarbamoyl, ethylthiocarbamoyl), C₆₋₁₄ aryl-carbamoyl (e.g., phenylcarbamoyl), C₃₋₁₀ cycloalkyl-carbamoyl (e.g., cyclopropylcarbamoyl), C₇₋₁₃ aralkyl-carbamoyl (e.g., benzylcarbamoyl), C₁₋₆ alkoxy-carbamoyl (e.g., methoxycarbamoyl), C₁₋₁₀ alkylsulfinyl group (e.g., methylsulfinyl, ethylsulfinyl), C₁₋₁₀ alkylsulfonyl group (e.g., methylsulfonyl, ethylsulfonyl), C₆₋₁₄ arylsulfonyl (e.g., phenylsulfonyl), (mono- or di-C₁₋₁₀ alkyl)phosphono group optionally forming a ring (e.g., dimethylphosphono; diethylphosphono; diisopropylphosphono; dibutylphosphono; 2-oxide-1,3,2-dioxaphosphinanyl), mono- or di-(C₁₋₆ alkyl optionally substituted by 1 to 3 halogens)-sulfamoyl (e.g., methylsulfamoyl, ethylsulfamoyl) and the like.

Examples of the "heterocyclic group" of the "optionally substituted heterocyclic group" exemplified as the substituents of ring A include those exemplified as the "heterocyclic group" of the aforementioned R⁴.

The heterocyclic group is preferably an azolyl group optionally fused with a benzene ring (e.g., pyrrolyl, imidazolyl, pyrazolyl, oxazolyl, isoxazolyl, thiazolyl, isothiazolyl, oxadiazolyl, thiadiazolyl, triazolyl, tetrazolyl) and the like.

The above-mentioned heterocyclic group optionally has 1 to 3 substituents at substitutable position(s). Examples of the substituents include an optionally substituted aliphatic hydrocarbon group, an optionally substituted alicyclic hydrocarbon group, an optionally substituted aromatic hydrocarbon group, an optionally substituted aromatic heterocyclic group, an optionally substituted nonaromatic heterocyclic group, a halogen atom, nitro, optionally substituted amino group, an optionally substituted hydroxy group, an optionally substituted mercapto group, an optionally substituted acyl group, C₁₋₃ alkylenedioxy, oxo and the like.

Here, examples of the "aliphatic hydrocarbon group", "alicyclic hydrocarbon group" and "aromatic hydrocarbon group" of the "optionally substituted aliphatic hydrocarbon group", "optionally substituted alicyclic hydrocarbon group" and "optionally substituted aromatic hydrocarbon group" include those exemplified as the "hydrocarbon group" of the "optionally substituted hydrocarbon group" exemplified as the substituents of ring A.

Examples of the substituent of the "aliphatic hydrocarbon group", "alicyclic hydrocarbon group" and "aromatic hydrocarbon group" include those exemplified as the "substituent" of the "optionally substituted hydrocarbon group" exemplified as the substituents of the ring A. The position of substitution and the number of substitution are not particularly limited. The number of substitution is preferably 1 to 3.

Examples of the "optionally substituted aromatic heterocyclic group" and "optionally substituted nonaromatic heterocyclic group" include those exemplified as the substituents of the "optionally substituted hydrocarbon group" exemplified as the substituents of ring A.

Examples of the "halogen atom", "optionally substituted amino group", "optionally substituted hydroxy group", "optionally substituted mercapto group", "optionally substituted acyl group" and "C₁₋₃ alkylenedioxy" include those exemplified as the substituents of the "optionally substituted hydrocarbon group" exemplified as the substituents of ring A.

Examples of the "optionally substituted hydroxy group", "optionally substituted mercapto group" and "optionally substituted amino group" exemplified as the substituents of ring A include those exemplified as the substituents of the "optionally substituted hydrocarbon group" exemplified as the substituents of ring A.

The substituent of ring A is preferably an optionally substituted hydrocarbon group, more preferably a C₁₋₁₀ alkyl group, a C₆₋₁₄ aryl group, a C₇₋₁₃ aralkyl group and the like. The substituent of ring A is particularly preferably a C₁₋₆ alkyl group (preferably methyl and the like).

Ring A is preferably imidazole, pyrazole, oxadiazole, thiadiazole, triazole or tetrazole ring (preferably pyrazole ring), each optionally having 1 or 2 substituents selected from a C₁₋₁₀ alkyl group, a C₆₋₁₄ aryl group and a C₇₋₁₃ aralkyl group (preferably C₁₋₆ alkyl group).

As the "optionally substituted hydrocarbon group" and "optionally substituted heterocyclic group" for B, those exemplified as the substituents of ring A are used. Here, the hydrocarbon group of the "optionally substituted hydrocarbon group" is preferably an alicyclic hydrocarbon group or an aromatic hydrocarbon group. In addition, the heterocyclic group of the "optionally substituted heterocyclic group" is preferably an aromatic heterocyclic group.

B is preferably an optionally substituted aromatic hydrocarbon group or an optionally substituted aromatic heterocyclic group.

B is more preferably an optionally substituted C₆₋₁₄ aryl group, an optionally substituted 5- to 7-membered monocyclic aromatic heterocyclic group and the like. Preferable specific examples of B include a C₆₋₁₄ aryl group (preferably phenyl) and a 5- to 7-membered monocyclic aromatic heterocyclic group (preferably furyl, thienyl, pyridyl, pyrimidinyl), each optionally having 1 to 3 substituents selected from C₁₋₆ alkyl optionally substituted by 1 to 3 halogen atoms, C₁₋₆ alkoxy optionally substituted by 1 to 3 halogen atoms and a halogen atom.

B is particularly preferably a C₆₋₁₄ aryl group (preferably phenyl) optionally having a halogen atom (preferably fluorine atom).

The "divalent noncyclic hydrocarbon group" for X may be linear or branched as long as it is a noncyclic divalent hydrocarbon group, and may be saturated or unsaturated.

Examples of the "divalent noncyclic hydrocarbon group" include "divalent aliphatic hydrocarbon group". Particularly, a divalent C₁₋₈ aliphatic hydrocarbon group exemplified below is preferable.
(1) C₁₋₈ alkylene (e.g., -CH₂-, -(CH₂)₂-, -(CH₂)₃-, -(CH₂)₄-, - (CH₂)₅-, -(CH₂)₆-, -(CH₂)₇-, -(CH₂)₈-, -CH(CH₃)-, -C(CH₃)₂-, - (CH(CH₃))₂-, - (CH₂)₂C(CH₃)₂₋, - (CH₂)₃C(CH₃)₂- and the like) ;
(2) C₂₋₈ alkenylene (e.g., -CH=CH-, -CH₂-CH=CH-, -C(CH₃)₂-CH=CH-, -CH₂-CH=CH-CH₂-, -CH₂-CH₂-CH=CH-, -CH=CH-CH=CH-, -CH=CH-CH₂-CH₂-CH₂- and the like) and the like.

The C₂₋₈ alkenylene encompasses both an E form and a Z form thereof.

The "divalent noncyclic hydrocarbon group" is preferably C₁₋₄ alkylene, C₂₋₄ alkenylene, more preferably -CH₂-, -(CH₂)₂-, - CH=CH- and the like. X is particularly preferably -CH=CH- and the like.

Z is -O-, -S-, -NR²-, -CONR²- or -NR²CO- (R² is a hydrogen atom or an optionally substituted alkyl group).

In the optionally substituted alkyl group for R², the alkyl group is, for example, a C₁₋₆ alkyl group. The alkyl group may have 1 to 3 substituents. Examples of the substituents include a halogen atom, C₁₋₆ alkoxy optionally substituted by 1 to 3 halogen atoms, hydroxy, nitro, amino and the like.

R² is preferably a hydrogen atom or a C₁₋₆ alkyl group, more preferably a hydrogen atom.

Z is preferably -CONR²- (R² is as defined above), more preferably -CONH- (in the present invention, the carbon atom (C) of -CONR²- is bonded to X, and a nitrogen atom (N) is bonded to Y).

Examples of the divalent noncyclic hydrocarbon group for Y include those exemplified as the aforementioned X.

Y is preferably a bond or C₁₋₄ alkylene, more preferably a bond, -CH₂-, -(CH₂)₂-, -(CH₂)₃- and the like. Y is particularly preferably a bond.

The cyclic group of the "optionally substituted cyclic group" for R¹ is, for example, an alicyclic hydrocarbon group, an aromatic hydrocarbon group, an aromatic heterocyclic group, a nonaromatic heterocyclic group and the like.

Here, examples of the "alicyclic hydrocarbon group" and "aromatic hydrocarbon group" include those exemplified as the "hydrocarbon group" of the "optionally substituted hydrocarbon group" exemplified as the substituents of ring A.

Examples of the "aromatic heterocyclic group" and "nonaromatic heterocyclic group" include those exemplified as the substituents of the "optionally substituted hydrocarbon group" exemplified as the substituents of ring A.

The cyclic group is preferably an optionally partially saturated C₆₋₁₄ aryl group (preferably phenyl, dihydroindenyl), a C₃₋₁₀ cycloalkyl group (preferably cyclopropyl, cyclobutyl, cyclopentyl, cyclohexyl, cycloheptyl, cyclooctyl), a C₃₋₁₀ cycloalkenyl group (preferably cyclohexenyl), a 5- or 6-membered aromatic heterocyclic group optionally fused with a benzene ring (preferably furyl, thienyl, oxazolyl, thiazolyl, isoxazolyl, imidazolyl, pyrazolyl, pyridyl, pyrazinyl, indolyl, quinolyl, isoquinolyl, benzothiadiazolyl), a 5- or 6-membered nonaromatic heterocyclic group optionally fused with a benzene ring (preferably pyrrolidinyl, tetrahydrofuranyl, thiazolinyl, oxazolinyl, thiazolidinyl, oxazolidinyl, dioxolanyl, piperidinyl, piperazinyl, morpholinyl, thiomorpholinyl, dihydrobenzofuranyl, oxodihydrobenzooxazolyl) and the like. The cyclic group is more preferably a C₆₋₁₄ aryl group, and phenyl is particularly preferable.

The "cyclic group" for R¹ optionally has 1 to 4 substituents at substitutable position(s). Examples of the substituents include
(1) nitro;
(2) oxo;
(3) hydroxy;
(4) cyano;
(5) a halogen atom;
(6) C₁₋₆ alkylenedioxy (e.g., methylenedioxy, ethylenedioxy);
(7) carboxyl;
(8) a C₁₋₆ alkyl group optionally substituted by 1 to 3 substituents selected from a halogen atom; hydroxy; cyano; C₁₋₆ alkoxy; amino optionally mono- or di-substituted by substituent(s) selected from C₁₋₆ alkyl and C₁₋₆ alkyl-carbonyl (e.g., amino, methylamino, dimethylamino, acetylamino, butyrylamino, isobutyrylamino, isovalerylamino); a 5- or 6-membered aromatic heterocyclic group (e.g., thiazolyl, oxazolyl, imidazolyl, pyrazolyl, oxadiazolyl, thiadiazolyl, triazolyl, tetrazolyl, pyridyl, pyrimidinyl)optionally substituted by 1 to 3 substituents selected from C₁₋₆ alkyl, hydroxy-C₁₋₆ alkyl, carboxyl, carbamoyl and C₁₋₆ alkoxy-carbonyl; an aromatic fused heterocyclic group (e.g., benzimidazolyl, benzopyrazolyl, benzothiazolyl, benzooxazolyl, benzotriazolyl, quinolyl, indazolyl) optionally substituted by 1 to 3 substituents selected from C₁₋₆ alkyl, hydroxy-C₁₋₆ alkyl, carboxyl, carbamoyl and C₁₋₆ alkoxy-carbonyl; a 5- or 6-membered nonaromatic heterocyclic group (e.g., tetrahydrofuryl, morpholinyl, thiomorpholinyl, piperidinyl, pyrrolidinyl, piperazinyl, dioxothiazolidinyl, dioxooxazolidinyl, oxodihydrooxadiazolyl, dioxoimidazolidinyl, dioxopiperazinyl, dioxidethiomorpholinyl) optionally substituted by 1 to 3 substituents selected from C₁₋₆ alkyl, hydroxy-C₁₋₆ alkyl, carboxyl, carbamoyl and C₁₋₆ alkoxy-carbonyl; a nonaromatic fused heterocyclic group (e.g., oxodihydrobenzooxazolyl, tetrahydrobenzothiazolyl) optionally substituted by 1 to 3 substituents selected from C₁₋₆ alkyl, hydroxy-C₁₋₆ alkyl, carboxyl, carbamoyl and C₁₋₆ alkoxy-carbonyl; a carboxyl group; C₁₋₆ alkoxy-carbonyl; a (mono- or di-C₁₋₁₀ alkyl) phosphono group optionally forming a ring (e.g., dimethylphosphono; diethylphosphono; diisopropylphosphono; dibutylphosphono; 2-oxide-1,3,2-dioxaphosphinanyl); carbamoyl optionally substituted by amino; mono- or di-C₁₋₆ alkyl-carbamoyl optionally substituted by 1 to 3 substituents selected from a halogen atom, hydroxy and C₁₋₆ alkoxy-carbonyl (e.g., methylcarbamoyl, ethylcarbamoyl, dimethylcarbamoyl, diethylcarbamoyl, ethylmethylcarbamoyl, propylcarbamoyl, trifluoroethylcarbamoyl, methoxycarbonylethylcarbamoyl, 2-hydroxy-1-methoxycarbonyl-ethylcarbamoyl, 2-hydroxy-1-methoxycarbonyl-propylcarbamoyl); mono- or di-C₆₋₁₄ aryl-carbamoyl optionally substituted by 1 to 3 substituents selected from optionally halogenated C₁₋₆ alkyl and C₁₋₆ alkoxy (e.g., phenylcarbamoyl, methoxyphenylcarbamoyl, trifluoromethylphenylcarbamoyl); mono- or di-C₇₋₁₃ aralkyl-carbamoyl optionally substituted by 1 to 3 substituents selected from amino optionally mono- or di-substituted by C₁₋₆ alkyl, optionally halogenated C₁₋₆ alkyl, hydroxy and C₁₋₆ alkoxy-carbonyl (e.g., benzylcarbamoyl, phenethylcarbamoyl, dimethylaminobenzylcarbamoyl, methoxycarbonylphenethylcarbamoyl, trifluoromethylbenzylcarbamoyl); sulfamoyl; optionally halogenated mono- or di-C₁₋₆ alkylsulfamoyl; C₁₋₆ alkylthio; C₁₋₆ alkylsulfinyl; C₁₋₆ alkylsulfonyl; C₁₋₆ alkyl-carbonyl; mono- or di-C₆₋₁₄ aryl-carboxamide (e.g., phenylcarboxamide); C₆₋₁₄ arylthio (e.g., phenylthio); 5- or 6-membered aromatic heterocyclethio (e.g., triazolylthio, tetrazolylthio) optionally substituted by C₁₋₆ alkyl; C₆₋₁₄ arylsulfinyl (e.g., phenylsulfinyl); 5- or 6-membered aromatic heterocyclylsulfinyl (e.g., triazolylsulfinyl, tetrazolylsulfinyl) optionally substituted by C₁₋₆ alkyl; C₆₋₁₄ arylsulfonyl (e.g., phenylsulfonyl); and 5- or 6-membered aromatic heterocyclylsulfonyl (e.g., triazolylsulfonyl, tetrazolylsulfonyl) optionally substituted by C₁₋₆ alkyl;
(9) a C₃₋₁₀ cycloalkyl group optionally substituted by 1 to 3 halogen atoms;
(10) C₆₋₁₄ aryl (e.g., phenyl) optionally substituted by 1 to 3 halogen atoms;
(11) C₇₋₁₃ aralkyl optionally substituted by 1 to 3 substituents selected from a halogen atom and hydroxy;
(12) a 5- or 6-membered aromatic heterocyclic group (e.g., thiadiazolyl, imidazolyl) optionally substituted by 1 to 3 substituents selected from a halogen atom, C₁₋₆ alkyl, and C₆₋₁₄ aryl (e.g., phenyl) optionally substituted by 1 to 3 halogen atoms;
(13) a C₁₋₆ alkoxy group optionally substituted by 1 to 3 substituents selected from a halogen atom; a 5- or 6-membered aromatic heterocyclic group (e.g., thiazolyl, oxazolyl, imidazolyl, pyrazolyl, oxadiazolyl, thiadiazolyl, triazolyl, tetrazolyl, pyridyl, pyrimidinyl) optionally substituted by 1 to 3 substituents selected from C₁₋₆ alkyl, hydroxy-C₁₋₆ alkyl, carboxyl, carbamoyl and C₁₋₆ alkoxy-carbonyl; an aromatic fused heterocyclic group (e.g., benzimidazolyl, benzopyrazolyl, benzothiazolyl, benzooxazolyl, benzotriazolyl, quinolyl, indazolyl) optionally substituted by 1 to 3 substituents selected from C₁₋₆ alkyl, hydroxy-C₁₋₆ alkyl, carboxyl, carbamoyl and C₁₋₆ alkoxy-carbonyl; a 5- or 6-membered nonaromatic heterocyclic group (e.g., tetrahydrofuryl, morpholinyl, thiomorpholinyl, piperidinyl, pyrrolidinyl, piperazinyl, dioxothiazolidinyl, dioxooxazolidinyl, oxodihydrooxadiazolyl, dioxoimidazolidinyl, dioxopiperazinyl, dioxidethiomorpholinyl) optionally substituted by 1 to 3 substituents selected from C₁₋₆ alkyl, hydroxy-C₁₋₆ alkyl, carboxyl, carbamoyl and C₁₋₆ alkoxy-carbonyl; and a nonaromatic fused heterocyclic group (e.g., oxodihydrobenzooxazolyl, tetrahydrobenzothiazolyl) optionally substituted by 1 to 3 substituents selected from C₁₋₆ alkyl, hydroxy-C₁₋₆ alkyl, carboxyl, carbamoyl and C₁₋₆ alkoxy-carbonyl;
(14) C₁₋₆ alkylthio optionally substituted by 1 to 3 substituents selected from a 5- or 6-membered aromatic heterocyclic group (e.g., thiazolyl, oxazolyl, triazolyl, tetrazolyl, pyridyl, pyrimidinyl) optionally substituted by C₁₋₆ alkyl, and a halogen atom;
(15) C₆₋₁₄ aryloxy (e.g., phenoxy) optionally substituted by 1 to 3 halogen atoms;
(16) amino optionally mono- or di-substituted by C₁₋₆ alkyl (e.g., amino, methylamino, dimethylamino, ethylamino, diethylamino, ethylmethylamino, propylamino, dibutylamino);
(17) phosphono-C₁₋₆ alkylamino optionally mono- or di-substituted by C₁₋₁₀ alkyl (e.g., phosphonomethylamino, diethylphosphonomethylamino);
(18) mono- or di-C₁₋₆ alkyl-carboxamide optionally substituted by 1 to 6, preferably 1 to 3, substituents selected from a halogen atom, C₁₋₆ alkyl and C₁₋₆ alkoxy-carbonyl (e.g., acetylamino, hexanoylamino, trifluoroacetylamino, N-acetyl-N-methylamino, pentafluoropropionylamino, ethoxycarbonylpropionylamino);
(19) a (mono- or di-C₁₋₁₀ alkyl)phosphono group optionally forming a ring (e.g., dimethylphosphono; diethylphosphono; diisopropylphosphono; dibutylphosphono; 2-oxide-1,3,2-dioxaphosphinanyl);
(20) C₁₋₆ alkyl-carbonyl optionally substituted by 1 to 3 halogen atoms;
(21) C₁₋₆ alkyl-sulfinyl optionally substituted by a 5- or 6-membered aromatic heterocyclic group (e.g., thiazolyl, oxazolyl, triazolyl, tetrazolyl, pyridyl, pyrimidinyl) optionally substituted by C₁₋₆ alkyl;
(22) C₁₋₆ alkyl-sulfonyl optionally substituted by a 5- or 6-membered aromatic heterocyclic group (e.g., thiazolyl, oxazolyl, triazolyl, tetrazolyl, pyridyl, pyrimidinyl) optionally substituted by C₁₋₆ alkyl;
(23) C₃₋₁₀ cycloalkyl-carbonyl optionally substituted by 1 to 3 halogen atoms;
(24) C₆₋₁₄ aryl-carbonyl (e.g., benzoyl) optionally substituted by 1 to 3 halogen atoms;
(25) C₇₋₁₃ aralkyl-carbonyl optionally substituted by 1 to 3 halogen atoms;
(26) C₆₋₁₄ aryl-C₂₋₆alkenyl-carbonyl (e.g., styrylcarbonyl) optionally substituted by 1 to 3 halogen atoms;
(27) C₁₋₆ alkoxy-carbonyl optionally substituted by 1 to 3 halogen atoms;
(28) 5- or 6-membered aromatic heterocyclyl-carbonyl (e.g., furoyl, pyrrolylcarbonyl, pyridylcarbonyl) optionally substituted by C₁₋₆ alkyl;
(29) 5- or 6-membered non-aromatic heterocyclyl-carbonyl (e.g., tetrahydrofuroyl) optionally substituted by C₁₋₆ alkyl;
(30) carbamoyl optionally mono- or di-substituted by C₁₋₆ alkyl (e.g., carbamoyl, dimethylcarbamoyl);
(31) sulfamoyl optionally substituted by 1 or 2 substituent selected from a 5- or 6-membered aromatic heterocyclic group (e.g., thiazolyl, oxazolyl, triazolyl, tetrazolyl, pyridyl, pyrimidinyl) optionally substituted by C₁₋₆ alkyl and C₁₋₆ alkyl (e.g., sulfamoyl, dimethylsulfamoyl);
(32) C₇₋₁₃ aralkyloxy-carbonylthio (e.g., benzyloxycarbonylthio, phenethyloxycarbonylthio) optionally substituted by 1 to 3 halogen atoms;
(33) C₁₋₆ alkoxy-carboxamide (e.g., tert-butoxy carboxamide);
(34) C₆₋₁₄ aryl-sulfonyl (e.g., phenylsulfonyl) optionally substituted by 1 to 3 halogen atoms; and the like.

The substituent of the above-mentioned "cyclic group" is preferably a C₁₋₆ alkyl group optionally substituted by 1 to 3 substituents selected from a 5- or 6-membered aromatic heterocyclic group (e.g., thiazolyl, oxazolyl, imidazolyl, pyrazolyl, oxadiazolyl, thiadiazolyl, triazolyl, tetrazolyl, pyridyl, pyrimidinyl) optionally substituted by C₁₋₆ alkyl; an aromatic fused heterocyclic group (e.g., benzimidazolyl, benzopyrazolyl, benzothiazolyl, benzooxazolyl, benzotriazolyl, quinolyl, indazolyl) optionally substituted by C₁₋₆ alkyl; a 5- or 6-membered nonaromatic heterocyclic group (e.g., tetrahydrofuryl, morpholinyl, thiomorpholinyl, piperidinyl, pyrrolidinyl, piperazinyl, dioxothiazolidinyl, dioxooxazolidinyl, oxodihydrooxadiazolyl, dioxoimidazolidinyl, dioxopiperazinyl, dioxidethiomorpholinyl) optionally substituted by C₁₋₆ alkyl; a (mono- or di-C₁₋₁₀ alkyl) phosphono group optionally forming a ring (e.g., dimethylphosphono; diethylphosphono; diisopropylphosphono; dibutylphosphono; 2-oxide-1,3,2-dioxaphosphinanyl); C₁₋₆ alkylsulfinyl; and C₁₋₆ alkylsulfonyl.

As the "optionally substituted amino group" and "optionally substituted acyl group" for R¹, those exemplified as the substituents of the "optionally substituted hydrocarbon group" exemplified as the substituent of ring A are used.

R¹ is preferably an optionally substituted cyclic group, more preferably a C₆₋₁₄ aryl group (preferably phenyl) optionally substituted by C₁₋₆ alkyl group optionally substituted by 1 to 3 substituents selected from a 5- or 6-membered aromatic heterocyclic group (e.g., thiazolyl, oxazolyl, imidazolyl, pyrazolyl, oxadiazolyl, thiadiazolyl, triazolyl, tetrazolyl, pyridyl, pyrimidinyl) optionally substituted by C₁₋₆ alkyl; an aromatic fused heterocyclic group (e.g., benzimidazolyl, benzopyrazolyl, benzothiazolyl, benzoxazolyl, benzotriazolyl, quinolyl, indazolyl) optionally substituted by C₁₋₆ alkyl; a 5- or 6-membered nonaromatic heterocyclic group (e.g., tetrahydrofuryl, morpholinyl, thiomorpholinyl, piperidinyl, pyrrolidinyl, piperazinyl, dioxothiazolidinyl, dioxooxazolidinyl, oxodihydrooxadiazolyl, dioxoimidazolidinyl, dioxopiperazinyl, dioxidethiomorpholinyl) optionally substituted by C₁₋₆ alkyl; a (mono- or di-C₁₋₁₀ alkyl) phosphono group optionally forming a ring (e.g., dimethylphosphono; diethylphosphono; diisopropylphosphono; dibutylphosphono; 2-oxide-1,3,2-dioxaphosphinanyl); C₁₋₆ alkylsulfinyl; and C₁₋₆ alkylsulfonyl.

The optionally substituted cyclic group for R¹ is preferably a group represented by the formula: 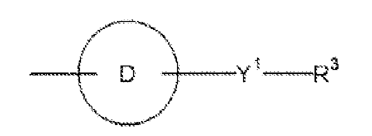 wherein D is a ring optionally further having substituent(s);
Y¹ is a bond or a divalent noncyclic hydrocarbon group; and R³ is an optionally substituted acyl group or an optionally substituted heterocyclic group, is preferable.

As the divalent noncyclic hydrocarbon group for Y¹, those exemplified as the aforementioned X can be mentioned.

Y¹ is preferably a bond or C₁₋₄ alkylene, more preferably a bond, -CH₂-, -(CH₂)₂-, - (CH₂)₃- and the like.

The ring of the "ring optionally further having substituent(s)" for D is, for example, a ring corresponding to the aforementioned "cyclic group" for R¹.

The ring for D is preferably C₆₋₁₄ aromatic hydrocarbon ring (preferably benzene, dihydroinden) optionally partially substituted, C₃₋₁₀ cycloalkane (preferably cyclopropane, cyclobutane, cyclopentane, cyclohexane, cycloheptane, cyclooctane), C₃₋₁₀ cycloalkene (preferably cyclohexene), a 5- or 6-membered aromatic heterocycle (preferably furan, thiophene, oxazole, thiazole, isoxazole, imidazole, pyrazole, pyridine, pyrazine, indole, quinoline, isoquinoline, benzothiadiazole) optionally fused with a benzene ring, a 5- or 6-membered non-aromatic heterocycle (preferably pyrrolidine, tetrahydrofuran, thiazoline, oxazoline, thiazolidine, oxazolidine, dioxolane, piperidine, piperazine, morpholine, thiomorpholine, dihydrobenzofuran, oxodihydrobenzooxazole) optionally fused with a benzene ring and the like. The above-mentioned ring is more preferably C₆₋₁₄ aromatic hydrocarbon, and benzene is particularly preferable.

The above-mentioned ring may have 1 to 3 substituents at substitutable position(s). Examples of the substituents include a halogen atom, a C₁₋₆ alkyl group optionally substituted by 1 to 3 halogen atoms, a C₁₋₆ alkoxy group optionally substituted by 1 to 3 halogen atoms and the like.

Examples of the "optionally substituted acyl group" for R³ include those exemplified as the substituents of the "optionally substituted hydrocarbon group" exemplified as the substituents of ring A. Examples of the "optionally substituted heterocyclic group" for R³ include those exemplified as the substituents of ring A.

Preferable examples of the "optionally substituted acyl group" for R³ include a carboxyl group; C₁₋₆ alkoxy-carbonyl; a (mono- or di-C₁₋₁₀ alkyl)phosphono group optionally forming a ring (e.g., dimethylphosphono; diethylphosphono; diisopropylphosphono; dibutylphosphono; 2-oxide-1,3,2-dioxaphosphinanyl); carbamoyl optionally substituted by amino; mono- or di-C₁₋₆ alkyl-carbamoyl optionally substituted by 1 to 3 substituents selected from a halogen atom, hydroxy and C₁₋₆ alkoxy-carbonyl (e.g., methylcarbamoyl, ethylcarbamoyl, dimethylcarbamoyl, diethylcarbamoyl, ethylmethylcarbamoyl, propylcarbamoyl, trifluoroethylcarbamoyl, methoxycarbonylethylcarbamoyl, 2-hydroxy-1-methoxycarbonylethylcarbamoyl, 2-hydroxy-1-methoxycarbonyl-propylcarbamoyl); mono- or di-C₆₋₁₄ aryl-carbamoyl optionally substituted by 1 to 3 substituents selected from optionally halogenated C₁₋₆ alkyl and C₁₋₆ alkoxy (e.g., phenylcarbamoyl, methoxyphenylcarbamoyl, trifluoromethylphenylcarbamoyl); mono- or di-C₇₋₁₃ aralkyl-carbamoyl optionally substituted by 1 to 3 substituents selected from amino optionally mono- or di-substituted by C₁₋₆ alkyl, optionally halogenated C₁₋₆ alkyl, hydroxy and C₁₋₆ alkoxy-carbonyl (e.g., benzylcarbamoyl, phenethylcarbamoyl, dimethylaminobenzylcarbamoyl, methoxycarbonylphenethylcarbamoyl, trifluoromethylbenzylcarbamoyl); sulfamoyl; optionally halogenated mono- or di-C₁₋₆ alkylsulfamoyl; C₁₋₆ alkylsulfinyl; C₁₋₆ alkylsulfonyl; C₁₋₆ alkyl-carbonyl; 5- or 6-membered aromatic heterocyclylsulfinyl (e.g., triazolylsulfinyl, tetrazolylsulfinyl) optionally substituted by C₁₋₆ alkyl; C₆₋₁₄ arylsulfonyl (e.g., phenylsulfonyl); 5- or 6-membered aromatic heterocyclylsulfonyl (e.g., triazolylsulfonyl, tetrazolylsulfonyl) optionally substituted by C₁₋₆ alkyl; and the like.

The "optionally substituted acyl group" for R³ is preferably a group represented by the formula: -SO₂R⁴, -SOR⁴ or -PO₃R⁴R⁵ wherein R⁴ and R⁵ are the same or different and each is a hydrogen atom, a hydrocarbon group or a heterocyclic group, and R⁴ and R⁵ may form a heterocycle together with the adjacent oxo-substituted phosphorus atom and two oxygen atoms.

The "optionally substituted acyl group" for R³ is particularly preferably C₁₋₆ alkylsulfonyl; or a (mono- or di-C₁₋₁₀ alkyl) phosphono group optionally forming a ring (e.g., dimethylphosphono; diethylphosphono; diisopropylphosphono; dibutylphosphono; 2-oxide-1,3,2-dioxaphosphinanyl).

Preferable examples of the "optionally substituted heterocyclic group" for R³ include a 5- or 6-membered aromatic heterocyclic group (e.g., thiazolyl, oxazolyl, imidazolyl, pyrazolyl, oxadiazolyl, thiadiazolyl, triazolyl, tetrazolyl, pyridyl, pyrimidinyl); an aromatic fused heterocyclic group (e.g., benzimidazolyl, benzopyrazolyl, benzothiazolyl, benzooxazolyl, benzotriazolyl, quinolyl, indazolyl); a 5- or 6-membered nonaromatic heterocyclic group (e.g., tetrahydrofuryl, morpholinyl, thiomorpholinyl, piperidinyl, pyrrolidinyl, piperazinyl, dioxothiazolidinyl, dioxooxazolidinyl, oxodihydrooxadiazolyl, dioxoimidazolidinyl, dioxopiperazinyl, dioxidethiomorpholinyl); and a nonaromatic fused heterocyclic group (e.g., oxodihydrobenzooxazolyl, tetrahydrobenzothiazolyl), which are each optionally substituted by 1 to 3 substituents selected from C₁₋₆ alkyl, hydroxy-C₁₋₆ alkyl, carboxyl, carbamoyl and C₁₋₆ alkoxy-carbonyl.

Among these, a 5- or 6-membered aromatic heterocyclic group (e.g., thiazolyl, oxazolyl, imidazolyl, pyrazolyl, oxadiazolyl, thiadiazolyl, triazolyl, tetrazolyl, pyridyl, pyrimidinyl), an aromatic fused heterocyclic group (e.g., benzimidazolyl, benzopyrazolyl, benzothiazolyl, benzooxazolyl, benzotriazolyl, quinolyl, indazolyl) and a 5- or 6-membered nonaromatic heterocyclic group (e.g., tetrahydrofuranyl, morpholinyl, thiomorpholinyl, piperidinyl, pyrrolidinyl, piperazinyl, dioxothiazolidinyl, dioxooxazolidinyl, oxodihydrooxadiazolyl, dioxoimidazolidinyl, dioxopiperazinyl, dioxidethiomorpholinyl), which are each optionally substituted by C₁₋₆ alkyl, are preferable.

The compound represented by the formula (I) is preferably a compound represented by the following formula (II): 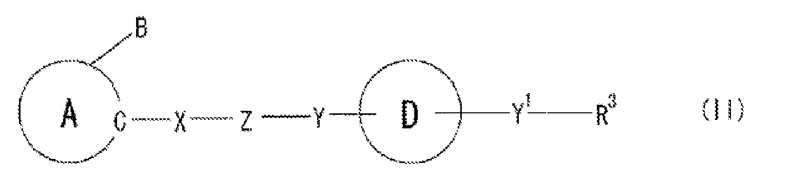 wherein ring A is a 5-membered aromatic heterocycle containing two or more nitrogen atoms and optionally further having substituent(s);
B is an optionally substituted hydrocarbon group or an optionally substituted heterocyclic group;
X is a divalent noncyclic hydrocarbon group;
Z is -O-, -S-, -NR²-, -CONR²- or -NR²CO- wherein R² is a hydrogen atom or an optionally substituted alkyl group;
Y and Y¹ are the same or different and each is a bond or a divalent noncyclic hydrocarbon group;
D is a ring optionally further having substituent(s); and
R³ is an optionally substituted acyl group or an optionally substituted heterocyclic group.

Preferable examples of the compound represented by the formula (II) include the following compounds.

### [Compound A]

A compound wherein
ring A is an imidazole, pyrazole, oxadiazole, thiadiazole, triazole or tetrazole ring (preferably pyrazole ring), each of which may have 1 or 2 substituents selected from a C₁₋₁₀ alkyl group, a C₆₋₁₄ aryl group and a C₇₋₁₃ aralkyl group (preferably C₁₋₆ alkyl group);
B is a C₆₋₁₄ aryl group (preferably phenyl) or a 5- to 7-membered monocyclic aromatic heterocyclic group (preferably furyl, thienyl, pyridyl, pyrimidinyl), each of which optionally has 1 to 3 substituents selected from C₁₋₆ alkyl optionally substituted by 1 to 3 halogen atoms, C₁₋₆ alkoxy optionally substituted by 1 to 3 halogen atoms and a halogen atom; more preferably a C₆₋₁₄ aryl group (preferably phenyl) optionally having a halogen atom (preferably fluorine atom);
X is C₁₋₄ alkylene or C₂₋₄ alkenylene; more preferably -CH₂-, -(CH₂)₂- or -CH=CH-; particularly preferably -CH=CH-;
Z is -CONR²- wherein R² is as defined above, a carbon atom (C) of -CONR²- is bonded to X, and a nitrogen atom (N) is bonded to Y; more preferably -CONH-;
Y is a bond or C₁₋₄ alkylene; more preferably a bond, -CH₂-, -(CH₂)₂- or - (CH₂)₃-; particularly preferably a bond;
Y¹ is a bond or C₁₋₄ alkylene; more preferably a bond, -CH₂-, -(CH₂)₂- or -(CH₂)₃-;
D is a C₆₋₁₄ aromatic hydrocarbon (preferably, benzene) optionally having 1 to 3 substituents selected from a halogen atom, a C₁₋₆ alkyl group optionally substituted by 1 to 3 halogen atoms and a C₁₋₆ alkoxy group optionally substituted by 1 to 3 halogen atoms; and
R³ is a carboxyl group; C₁₋₆ alkoxy-carbonyl; a (mono- or di-C₁₋₁₀ alkyl)phosphono group optionally forming a ring (e.g., dimethylphosphono; diethylphosphono; diisopropylphosphono; dibutylphosphono; 2-oxide-1,3,2-dioxaphosphinanyl); carbamoyl optionally substituted by amino; mono- or di-C₁₋₆ alkyl-carbamoyl optionally substituted by 1 to 3 substituents selected from a halogen atom, hydroxy and C₁₋₆ alkoxy-carbonyl; mono- or di-C₆₋₁₄ aryl-carbamoyl optionally substituted by 1 to 3 substituents selected from optionally halogenated C₁₋₆ alkyl and C₁₋₆ alkoxy; mono- or di-C₇₋₁₃ aralkyl-carbamoyl optionally substituted by 1 to 3 substituents selected from amino optionally mono- or di-substituted by C₁₋₆ alkyl, optionally halogenated C₁₋₆ alkyl, hydroxy and C₁₋₆ alkoxy-carbonyl; sulfamoyl; optionally halogenated mono- or di-C₁₋₆ alkylsulfamoyl; C₁₋₆ alkylsulfinyl; C₁₋₆ alkylsulfonyl; C₁₋₆ alkyl-carbonyl; 5- or 6-membered aromatic heterocyclylsulfinyl (e.g., triazolylsulfinyl, tetrazolylsulfinyl) optionally substituted by C₁₋₆ alkyl; C₆₋₁₄ arylsulfonyl (e.g., phenylsulfonyl); or 5- or 6-membered aromatic heterocyclylsulfonyl (e.g., triazolylsulfonyl, tetrazolylsulfonyl) optionally substituted by C₁₋₆ alkyl; more preferably C₁₋₆ alkylsulfonyl; a (mono- or di-C₁₋₁₀ alkyl)phosphono group optionally forming a ring (e.g., dimethylphosphono; diethylphosphono; diisopropylphosphono; dibutylphosphono; or 2-oxide-1,3,2-dioxaphosphinanyl).

### [Compound B]

A compound wherein
ring A is an imidazole, pyrazole, oxadiazole, thiadiazole, triazole or tetrazole ring (preferably pyrazole ring), each of which may have 1 or 2 substituents selected from a C₁₋₁₀ alkyl group, a C₆₋₁₄ aryl group and a C₇₋₁₃ aralkyl group (preferably C₁₋₆ alkyl group);
B is a C₆₋₁₄ aryl group (preferably phenyl) or a 5- to 7-membered monocyclic aromatic heterocyclic group (preferably furyl, thienyl, pyridyl, pyrimidinyl), each of which optionally has 1 to 3 substituents selected from C₁₋₆ alkyl optionally substituted by 1 to 3 halogen atoms, C₁₋₆ alkoxy optionally substituted by 1 to 3 halogen atoms and a halogen atom; more preferably a C₆₋₁₄ aryl group (preferably phenyl) optionally having a halogen atom (preferably fluorine atom);
X is C₁₋₄ alkylene or C₂₋₄ alkenylene; more preferably -CH₂-, -(CH₂)₂- or -CH=CH-; particularly preferably -CH=CH-;
Z is -CONR²- wherein R² is as defined above, a carbon atom (C) of -CONR²- is bonded to X, and a nitrogen atom (N) is bonded to Y; more preferably -CONH-;
Y is a bond or C₁₋₄ alkylene; more preferably a bond, -CH₂-, -(CH₂)₂- or - (CH₂)₃-; particularly preferably a bond;
Y¹ is a bond or C₁₋₄ alkylene; more preferably a bond, - CH₂-, -(CH₂)₂- or - (CH₂)₃-;
D is a C₆₋₁₄ aromatic hydrocarbon (preferably, benzene) optionally having 1 to 3 substituents selected from a halogen atom, a C₁₋₆ alkyl group optionally substituted by 1 to 3 halogen atoms and a C₁₋₆ alkoxy group optionally substituted by 1 to 3 halogen atoms; and
R³ is a 5- or 6-membered aromatic heterocyclic group (e.g., thiazolyl, oxazolyl, imidazolyl, pyrazolyl, oxadiazolyl, thiadiazolyl, triazolyl, tetrazolyl, pyridyl, pyrimidinyl); an aromatic fused heterocyclic group (e.g., benzimidazolyl, benzopyrazolyl, benzothiazolyl, benzooxazolyl, benzotriazolyl, quinolyl, indazolyl); a 5- or 6-membered nonaromatic heterocyclic group (e.g., tetrahydrofuryl, morpholinyl, thiomorpholinyl, piperidinyl, pyrrolidinyl, piperazinyl, dioxothiazolidinyl, dioxooxazolidinyl, oxodihydrooxadiazolyl, dioxoimidazolidinyl, dioxopiperazinyl, dioxidethiomorpholinyl); or a nonaromatic fused heterocyclic group (e.g., oxodihydrobenzooxazolyl, tetrahydrobenzothiazolyl), which are each optionally substituted by 1 to 3 substituents selected from C₁₋₆ alkyl, hydroxy-C₁₋₆ alkyl, carboxyl, carbamoyl and C₁₋₆ alkoxy-carbonyl; more preferably a 5- or 6-membered aromatic heterocyclic group (e.g., thiazolyl, oxazolyl, imidazolyl, pyrazolyl, oxadiazolyl thiadiazolyl, triazolyl, tetrazolyl, pyridyl, pyrimidinyl), an aromatic fused heterocyclic group (e.g., benzimidazolyl, benzopyrazolyl, benzothiazolyl, benzooxazolyl, benzotriazolyl, quinolyl, indazolyl), or a 5- or 6-membered nonaromatic heterocyclic group (e.g., tetrahydrofuranyl, morpholinyl, thiomorpholinyl, piperidinyl, pyrrolidinyl, piperazinyl, dioxothiazolidinyl, dioxooxazolidinyl, oxodihydrooxadiazolyl, dioxoimidazolidinyl, dioxopiperazinyl, dioxidethiomorpholinyl) which are each optionally substituted by C₁₋₆ alkyl.

### [Compound C]

diethyl [4-({(2E)-3-[5-(4-fluorophenyl)-1-methyl-1H-pyrazol-4-yl]prop-2-enoyl}amino)benzyl]phosphate [hereinafter sometimes to be abbreviated as "compound (a)"];
(2E)-N-{4-[(2,4-dioxo-1,3-thiazolidin-5-yl)methyl]phenyl}-3-[5-(4-fluorophenyl)-1-methyl-1H-pyrazol-4-yl]acrylamide [hereinafter sometimes to be abbreviated as "compound (b)"] ;
(2E)-3-[5-(4-fluorophenyl)-1-methyl-1H-pyrazol-4-yl]-N-[4-(1H-imidazol-1-ylmethyl)phenyl]acrylamide [hereinafter sometimes to be abbreviated as "compound (c)"];
(2E)-3-[5-(4-fluorophenyl)-1-methyl-1H-pyrazol-4-yl]-N-[4-(1H-pyrazol-1-ylmethyl)phenyl]acrylamide [hereinafter sometimes to be abbreviated as "compound (d)"]; diethyl [4-({(2E)-3-[1-methyl-5-(2-thienyl)-1H-pyrazol-4-yl]prop-2-enoyl}amino)benzyl]phosphate [hereinafter sometimes to be abbreviated as "compound (e)"];
(2E)-3-[5-(4-fluorophenyl)-1-methyl-1H-pyrazol-4-yl]-N-{4-[(3-methyl-2,4-dioxo-1,3-thiazolidin-5-yl)methyl]phenyl}acrylamide [hereinafter sometimes to be abbreviated as "compound (f)"];
(2E)-N-[4-(1H-benzimidazol-1-ylmethyl)phenyl]-3-[5-(4-fluorophenyl)-1-methyl-1H-pyrazol-4-yl]acrylamide [hereinafter sometimes to be abbreviated as "compound (g)"];
(2E)-3-[5-(4-fluorophenyl)-1-methyl-1H-pyrazol-4-yl]-N-{4-[(methylsulfonyl)methyl]phenyl}acrylamide [hereinafter sometimes to be abbreviated as "compound (h)"];
(2E)-3-[5-(4-fluorophenyl)-1-methyl-1H-pyrazol-4-yl]-N-{4-[hydroxy(2-pyridinyl)methyl]phenyl}acrylamide [hereinafter sometimes to be abbreviated as "compound (i)"];
(2E)-3-[5-(4-fluorophenyl)-1-methyl-1H-pyrazol-4-yl]-N-[4-(4-morpholinylmethyl)phenyl]acrylamide [hereinafter sometimes to be abbreviated as "compound (j)"];
(2E)-N-{4-[(ethylsulfonyl)methyl]phenyl}-3-[5-(4-fluorophenyl)-1-methyl-1H-pyrazol-4-yl]acrylamide [hereinafter sometimes to be abbreviated as "compound (k)"]; and
(2E)-3-[5-(4-fluorophenyl)-1-methyl-1H-pyrazol-4-yl]-N-{4-[2-(1,3,4-oxadiazol-2-yl)ethyl]phenyl}acrylamide [hereinafter sometimes to be abbreviated as "compound (1)"].

As a salt of a compound represented by the formula (I), a pharmacologically acceptable salt is preferable. Examples of such salt include salts with inorganic base, a salt with organic base, a salt with inorganic acid, a salt with organic acid, a salt with basic or acidic amino acid and the like.

Preferable examples of salts with inorganic base include alkali metal salt such as sodium salt, potassium salt and the like; alkaline earth metal salt such as calcium salt, magnesium salt and the like; and aluminum salt, ammonium salt and the like.

Preferable examples of salts with organic base include salts with trimethylamine, triethylamine, pyridine, picoline, ethanolamine, diethanolamine, triethanolamine, dicyclohexylamine, N,N-dibenzylethylenediamine and the like.

Preferable examples of salts with inorganic acid include salts with hydrochloric acid, hydrobromic acid, nitric acid, sulfuric acid, phosphoric acid and the like.

Preferable examples of salts with organic acid include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid and the like.

Preferable examples of salts with basic amino acid include salts with arginine, lysine, ornithine and the like.

Preferable examples of salts with acidic amino acid include salts with aspartic acid, glutamic acid and the like.

A compound represented by the formula (I) or a salt thereof [to be sometimes abbreviated as compound (I) in the present specification] can be produced according to a method known per se. Examples of such method include the method described in WO 2004/039365 or a method analogous thereto and the like.

The compound (I) may be used as a prodrug. A prodrug of the compound (I) means a compound capable of being converted to the compound (I) with a reaction due to an enzyme, an gastric acid, etc. under the physiological condition in the living body, that is, a compound capable of being converted to the compound (I) upon enzymatic oxidation, reduction, hydrolysis, etc.; a compound capable of being converted to the compound (I) by hydrolysis etc. due to gastric acid, etc. A prodrug of compound (I) may be a compound obtained by subjecting an amino group in compound (I) to an acylation, alkylation or phosphorylation (e.g., a compound obtained by subjecting an amino group in compound (I) to an eicosanoylation, alanylation, pentylaminocarbonylation, (5-methyl-2-oxo-1,3-dioxolen-4-yl)methoxycarbonylation, tetrahydrofuranylation, pyrrolidylmethylation, pivaloyloxymethylation and tert-butylation); a compound obtained by subjecting a hydroxy group in compound (I) to an acylation, alkylation, phosphorylation or boration (e.g., a compound obtained by subjecting a hydroxy group in compound (I) to an acetylation, palmitoylation, propanoylation, pivaloylation, succinylation, fumarylation, alanylation and dimethylaminomethylcarbonylation); a compound obtained by subjecting a carboxyl group in compound (I) to an esterification or amidation (e.g., a compound obtained by subjecting a carboxy group in compound (I) to an ethyl esterification, phenyl esterification, carboxymethyl esterification, dimethylaminomethyl esterification, pivaloyloxymethyl esterification, ethoxycarbonyloxyethyl esterification, phthalidyl esterification, (5-methyl-2-oxo-1,3-dioxolen-4-yl)methyl esterification, cyclohexyloxycarbonylethyl esterification and methylamidation) and the like. Any of these compounds can be produced from compound (I) by a method known *per se*.

A prodrug for compound (I) may also be one which is converted into compound (I) under a physiological condition, such as those described in IYAKUHIN no KAIHATSU (Development of Pharmaceuticals), Vol.7, Design of Molecules, p.163-198, Published by HIROKAWA SHOTEN, 1990.

In addition, a compound may be labeled with an isotope (e.g., ³H, ¹⁴C, ³⁵S, ¹²⁵I etc.) and the like.

Furthermore, compound (I) may be anhydride or hydrate.

The ferrochelatase activator of the present invention is generally used as a pharmaceutical composition obtained by formulating "a substance obtained by the screening method of the present invention" or "compound (I)" and a pharmacologically acceptable carrier according to a method known per se (e.g., the method described in the Japanese Pharmacopoeia).

As pharmacologically acceptable carriers, various organic or inorganic carrier substances conventionally used as preparation materials can be used. Examples of the carriers include excipient, lubricant, binder, disintegrant for solid preparations; solvent, solubilizer, suspending agent, isotonizing agent, buffer, soothing agent for liquid preparations and the like. For formulation of a preparation, a preparation additive such as a preservative, an antioxidant, a colorant, a sweetener and the like can be used as necessary.

Preferable examples of the excipient include lactose, sucrose, D-mannitol, D-sorbitol, starch, gelatinized starch, dextrin, crystalline cellulose, low-substituted hydroxypropylcellulose, sodium carboxymethylcellulose, gum arabic, pullulan, light anhydrous silicic acid, synthetic aluminum silicate, magnesium alumino metasilicate, xylitol, sorbitol, erythritol and the like.

Preferable examples of the lubricant include magnesium stearate, calcium stearate, talc, colloidal silica, polyethylene glycol 6000 and the like.

Preferable examples of the binder include gelatinized starch, sucrose, gelatin, gum arabic, methylcellulose, carboxymethylcellulose, sodium carboxymethylcellulose, crystalline cellulose, sucrose, D-mannitol, trehalose, dextrin, pullulan, hydroxypropylcellulose, hydroxypropylmethylcellulose, polyvinylpyrrolidone and the like.

Preferable examples of the disintegrant include lactose, sucrose, starch, carboxymethylcellulose, calcium carboxymethylcellulose, croscarmellose sodium, sodium carboxymethyl starch, low-substituted hydroxypropylcellulose, light anhydrous silicic acid, calcium carbonate and the like.

Preferable examples of the solvent include water for injection, saline, Ringer's solution, alcohol, propylene glycol, polyethylene glycol, sesame oil, corn oil, olive oil, cottonseed oil and the like.

Preferable examples of the solubilizers include polyethylene glycol, propylene glycol, D-mannitol, trehalose, benzyl benzoate, ethanol, trisaminomethane, cholesterol, triethanolamine, sodium carbonate, sodium citrate, sodium salicylate, sodium acetate and the like.

Preferable examples of the suspending agent include surfactant such as stearyltriethanolamine, sodium lauryl sulfate, lauryl aminopropionic acid, lecithin, benzalkonium chloride, benzethonium chloride, glycerol monostearate and the like; hydrophilic polymer such as polyvinyl alcohol, polyvinylpyrrolidone, sodium carboxymethylcellulose, methylcellulose, hydroxymethylcellulose, hydroxyethylcellulose, hydroxypropylcellulose and the like; polysorbates, polyoxyethylene hydrogenated castor oil and the like.

Preferable examples of the isotonizing agent include sodium chloride, glycerol, D-mannitol, D-sorbitol, glucose, xylitol, fructose and the like.

Preferable examples of the buffering agent include buffer such as phosphate, acetate, carbonate, citrate etc. and the like.

Preferable examples of the soothing agent include propylene glycol, lidocain hydrochloride, benzyl alcohol and the like.

Preferable examples of the preservative include paraoxybenzoates, chlorobutanol, benzyl alcohol, phenethyl alcohol, dehydroacetic acid, sorbic acid and the like.

Preferable examples of the antioxidant include sulfite, ascorbate and the like.

Preferable examples of the colorant include aqueous colored tar pigment (e.g., food colors such as Food Color Red Nos. 2 and 3, Food Color Yellow Nos. 4 and 5, Food Color Blue Nos. 1 and 2 etc.), insoluble lake dye (e.g., aluminum salt of the aforementioned aqueous food tar color), natural dye (e.g., β-carotene, chlorophyll, red iron oxide) and the like.

Preferable examples of the sweetener include saccharin sodium, dipotassium glycyrrhizinate, aspartame, stevia and the like.

Examples of the dosage form of the aforementioned pharmaceutical composition include oral preparations such as tablet (including sublingual tablet, orally disintegrating tablet), capsule (including soft capsule, microcapsule), granule, powder, troche, syrup, emulsion, suspension and the like; and parenteral preparations such as injection (e.g., subcutaneous injection, intravenous injection, intramuscular injection, intraperitoneal injection, drip infusion), external preparation (e.g., dermal preparation, ointment etc.), suppository (e.g., rectal suppository, vaginal suppository), pellet, nasal preparations, pulmonary preparation (inhalant), eye drop and the like. These preparations may be a release controlled preparation (e.g., sustained-release microcapsule) such as an immediate-release preparation, a sustained-release preparation and the like.

The content of "a substance obtained the screening method of the present invention" or "compound (I)" in the above-mentioned pharmaceutical composition is, for example, 0.1 - 100 wt%.

The production methods of an oral preparation and a parenteral preparation are specifically explained in the following. The oral preparation is produced by adding, for example, an excipient (e.g., lactose, sucrose, starch, D-mannitol, xylitol, sorbitol, erythritol, crystalline cellulose, light anhydrous silicic acid), a disintegrant (e.g., calcium carbonate, starch, carboxymethylcellulose, calcium carboxymethylcellulose, low-substituted hydroxypropylcellulose, croscarmellose sodium, sodium carboxymethyl starch, light anhydrous silicic acid), a binder (e.g., gelatinized starch, gum arabic, carboxymethylcellulose, hydroxypropylcellulose, hydroxypropylmethylcellulose, polyvinylpyrrolidone, crystalline cellulose, methylcellulose, sucrose, D-mannitol, trehalose, dextrin), a lubricant (e.g., talc, magnesium stearate, calcium stearate, colloidal silica, polyethylene glycol 6000) and the like to the active ingredient, and compression molding the mixture.

In addition, an oral preparation may be coated by a method known per se for the purpose of masking the taste, enteric coating or sustained release coating. Examples of the coating agent include enteric polymers (e.g., cellulose acetate phthalate, methacrylic acid copolymer L, methacrylic acid copolymer LD, methacrylic acid copolymer S, hydroxypropylmethylcellulosephthalate, hydroxypropylmethylcelluloseacetatesuccinate, carboxymethylethylcellulose), gastrosoluble polymers (e.g., polyvinyl acetaldiethylaminoacetate, aminoalkylmethacrylate copolymer E), aqueous polymers (e.g., hydroxypropylcellulose, hydroxypropylmethylcellulose), water insoluble polymers (e.g., ethylcellulose, aminoalkylmethacrylate copolymer RS, ethyl acrylate or methacrylic acid methyl copolymer), wax and the like. For coating, plasticizers such as polyethylene glycol and the like; and light shielding agents such as titanium oxide, diiron trioxide and the like may be used together with the above-mentioned coating agents.

Injection is produced by dissolving, suspending or emulsifying an active ingredient in an aqueous solvent (e.g., distilled water, physiological brine, Ringer's solution) or an oleaginous solvent (e.g., vegetable oil such as olive oil, sesame oil, cottonseed oil, corn oil and the like; propylene glycol, macrogol, tricaprylin) and the like, together with a dispersant (e.g., Tween 80 (manufactured by Atlas Powder, USA), HCO 60 (manufactured by Nikko Chemicals), polyethylene glycol, carboxymethylcellulose, sodium alginate), a preservative (e.g., methylparaben, propylparaben, benzyl alcohol, chlorobutanol, phenol), an isotonizing agent (e.g., sodium chloride, glycerol, D-sorbitol, D-mannitol, xylitol, glucose, fructose) and the like. In this case, when desired, additives such as solubilizers (e.g., sodium salicylate, sodium acetate, polyethylene glycol, propylene glycol, D-mannitol, trehalose, benzyl benzoate, ethanol, trisaminomethane, cholesterol, triethanolamine, sodium carbonate, sodium citrate), suspending agents (e.g., surfactant such as stearyltriethanolamine, sodium lauryl sulfate, lauryl aminopropionic acid, lecithin, benzalkonium chloride, benzethonium chloride, glycerol monostearate and the like; hydrophilic polymers such as polyvinyl alcohol, polyvinylpyrrolidone, sodium carboxymethylcellulose, methylcellulose, hydroxymethylcellulose, hydroxyethylcellulose, hydroxypropylcellulose etc.), buffering agents (e.g., buffer such as phosphate, acetate, carbonate, citrate etc.), stabilizers (e.g., human serum albumin), soothing agents (e.g., propylene glycol, lidocain hydrochloride, benzyl alcohol), preservatives (e.g., paraoxybenzoates, chlorobutanol, benzalkonium chloride, benzyl alcohol, phenethyl alcohol, dehydroacetic acid, sorbic acid) and the like may be used.

External preparation is produced by processing an active ingredient into a solid, semisolid or liquid composition. For example, the above-mentioned solid composition is produced by using the active ingredient as it is, or adding an excipient (e.g., lactose, D-mannitol, starch, crystalline cellulose, sucrose), a thickener (e.g., natural rubbers, cellulose derivative, acrylic acid polymer) and the like thereto, mixing and powdering them. The above-mentioned liquid composition is produced in almost the same manner as in the injection. Semisolid composition is preferably an aqueous or oleaginous gel agent, or an ointment. These compositions may contain a pH regulator (e.g., phosphoric acid, citric acid, hydrochloric acid, sodium hydroxide), a preservative (e.g., paraoxybenzoates, chlorobutanol, benzalkonium chloride, benzyl alcohol, phenethyl alcohol, dehydroacetic acid, sorbic acid) and the like. Suppository is produced by processing the active ingredient into an oleaginous or aqueous, solid, semisolid or liquid composition. Examples of the oleaginous base to be used for the production of the composition include higher fatty acid glycerides [e.g., cacao butter, Witepsols], medium fatty acid triglyceride [e.g., miglyols], vegetable oil (e.g., sesame oil, soybean oil, cottonseed oil) and the like. Examples of the aqueous base include polyethylene glycols, propylene glycol and the like. In addition, examples of the aqueous gel base include natural rubbers, cellulose derivative, vinyl polymer, acrylic acid polymer and the like.

The dose of the ferrochelatase activator of the present invention can be appropriately determined according to the subject of administration, administration route, target disease, symptom and the like. The dose of the ferrochelatase activator of the present invention for oral administration to an adult patient is generally about 0.05 - 500 mg/kg body weight, preferably about 0.5 - 100 mg/kg body weight, as a single dose of "a substance obtained by the screening method of the present invention" or "compound (I)", which is the active ingredient. This amount is desirably administered in one to 3 portions a day.

For example, the dose of the ferrochelatase activator of the present invention for oral administration to an adult patient with diabetes or nerve system disease, mitochondrial encephalomyopathy, fibromyalgia, chronic fatigue syndrome, pancreatic exhaustion, steatohepatitis or anemia, showing a decreased mitochondrial function in a tissue, is generally about 0.05 - 50 mg/kg body weight, preferably about 0.2 - 4 mg/kg body weight, as a single dose of "a substance obtained by the screening method of the present invention" or "compound (I)", which is the active ingredient. This amount is desirably administered in one to 3 portions a day.

### Examples

The present invention is explained in more detail in the following by referring to Examples, which are not to be construed as limitative.

### Example 1: Evaluation of ferrochelatase activity and cytochrome c oxidase activity in streptozotocin-administrated diabetic rat

(1) A streptozotocin (Sigma S-0130) solution was injected to male SD rats (CLEA Japan, Inc.) via the tail vein to induce diabetes. In the following experiments, rats at one or two months after streptozotocin administration were used. The same experiment was performed without the streptozotocin administration, and the rats were used as a control group.
(2) The rats were subjected to thoracotomy under pentobarbital anesthesia, and saline was refluxed from the heart to allow exsanguination. Then, the spinal cord L4-5 region was separated from the rats, a tissue extract was prepared, and the ferrochelatase activity, cytochrome c oxidase activity and protein concentration were determined.
(3) The ferrochelatase activity was measured by reference to the method of RONG GUO et al. (Journal of Chromatography, vol. 566, No. 2, pp. 383-396, 1991). The spinal cord lysate was diluted with Tris-HCl buffer (pH 7.6) containing 0.001% Tween 80 and 50 or 100 nmol/L zinc acetate and added to a 96 well plate (fluoro B plate, Dainippon Pharmaceutical Co., Ltd.) for fluorescence measurement at 180 µL/well. Then, 0.5 or 1 µmol/L of protoporphyrin IX (ICN, catalog No.: ICN102757) was added to the well plate at 20 µL/well to start the reaction. The production of zinc-protoporphyrin IX was measured immediately after the start of the reaction or over time by determining at an excitation wavelength of 405 nm and a fluorescence wavelength of 590 nm. The change of fluorescence caused by the reaction for 20 min from the start of the reaction was corrected based on the protein amount of the tissue extract added, and the ferrochelatase activity was calculated per tissue.
(4) The cytochrome c oxidase activity was measured using Cytochrome c Oxidase Assay Kit (Sigma CYTO-OX1) according to the manual of the kit. The measurement value was corrected based on the protein amount of the tissue extract added to the reaction mixture, to calculate the cytochrome c oxidase activity per tissue.

Table 1 shows the ferrochelatase activity and cytochrome c oxidase activity in the rat-derived spinal cord tissue extract at one month from the streptozotocin administration. The numerical values in the Table show mean±standard deviation of n=5 (n is the number of rats).

Table 2 shows the ferrochelatase activity and cytochrome c oxidase activity of the rat-derived spinal cord tissue extract at two months from the streptozotocin administration. The numerical values in the Table show mean±standard deviation of n=8 (n is the number of rats).

**Table 1**

| One month after induction of diabetes by streptozotocin | | |
|---|---|---|
| | normal rat | streptozotocin induced diabetic rat |
| ferrochelatase activity (Em 590 nm/mg protein) | 6.50 ± 0.47 | 5.07 ± 0.37 ## |
| | | |
| cytochrome c oxidase activity (units/mg protein) | 146.1 ± 10.4 | 134.2 ± 11.2 |

| | | |
|---|---|---|
| ##: significant difference by t-test (p<0.01). | | |

**Table 2**

| Two months after induction of diabetes by streptozotocin | | |
|---|---|---|
| | normal rat | streptozotocin induced diabetic rat |
| ferrochelatase activity (Em 590 nm/mg protein) | 9.68 ± 0.85 | 6.07 ± 0.79 ## |
| | | |
| cytochrome c oxidase activity (units/mg protein) | 159.95 ± 13.27 | 100.7 ± 17.0 ## |

| | | |
|---|---|---|
| ##: significant difference by t-test (p<0.01). | | |

As is clear from Table 1, diabetic rat-derived spinal cord at one month after the streptozotocin administration showed a significant decrease in the ferrochelatase activity, but only a tendency toward decrease as for the cytochrome c oxidase activity.

As is clear from Table 2, moreover, diabetic rat-derived spinal cord at two months after the streptozotocin administration showed a significant decrease in the ferrochelatase activity and cytochrome c oxidase activity.

The above results show that, in the rat with streptozotocin-induced diabetes, the ferrochelatase activity decreases prior to a decrease in the cytochrome c oxidase activity, which is caused by the induced diabetes. This shows a possibility that a decrease in the ferrochelatase activity is involved in the onset of diabetic complications in streptozotocin-administrated diabetic rats.

### Example 2: Evaluation of ferrochelatase activity, cytochrome c oxidase activity and mitochondrial ATP production activity in Wistar Fatty rat

(1) Using Wistar Fatty rat and Lean rat (TAKEDA RABICS, 7-week-old), liver crude mitochondrial fractions were prepared.
(2) Crude liver mitochondrial fractions were prepared based on the method of Rolf Wibom et al. (American Journal of Physiology, vol. 259, pp. E204-209, 1990). After exsanguination by decapitating the rat, the liver (about 100 mg) was separated, and a total liver homogenate in 2 mL of buffer A (100 mmol/L potassium chloride, 50 mmol/L Tris-HCl buffer pH 7.5, 5 mmol/L magnesium chloride, 1.8 mmol/L disodium adenosine-5'-3 phosphate, 1 mmol/L EDTA) was prepared. The total liver homogenate (0.2 mL) was fractionated, centrifuged at 600 g×3 min, and the supernatant (1.5 mL) was collected and further centrifuged at 15000 g×3 min. The obtained precipitate was suspended in 0.5 mL of buffer B (180 mmol/L sucrose, 35 mmol/L potassium phosphate buffer pH 7.5, 10 mmol/L magnesium chloride, 1 mmol/L EDTA), centrifuged at 15000g×3 min and the obtained precipitate was suspended in buffer B (0.5 mL) to give a crude mitochondrial fraction. Using the crude mitochondrial fraction, the ferrochelatase activity, cytochrome c oxidase activity, atractyloside-sensitive mitochondrial ATP production activity and protein concentration were examined.
(3) The ferrochelatase activity was measured by reference to the method of RONG GUO et al. (mentioned above). The above-mentioned crude mitochondrial fraction was diluted with Tris-HCl buffer (pH 7.6) containing 0.01% Tween 80 and 100 µmol/L zinc acetate and added to a 96 well plate for fluorescence measurement at 180 µL/well. Then, 1 µmol/L of protoporphyrin IX was added to the well plate at 20 µL/well to start the reaction. The production of zinc-protoporphyrin IX was measured immediately after the start of the reaction or over time by determining at an excitation wavelength of 405 nm and a fluorescence wavelength of 590 nm. The change of fluorescence caused by the reaction for 20 min from the start of the reaction was amended based on the protein amount of the crude mitochondrial fraction added, and the ferrochelatase activity was calculated per mitochondria.
(4) The cytochrome c oxidase activity was measured using Cytochrome c Oxidase Assay Kit according to the manual of the kit. The measurement value was amended based on the protein amount of the crude mitochondrial fraction added to the reaction mixture, and the cytochrome c oxidase activity was calculated per mitochondria.
(5) The mitochondrial ATP production activity was measured based on the method of Rolf Wibom et al. (mentioned above). The above-mentioned crude mitochondrial fraction was 100-fold diluted with buffer B, 10 µL thereof was collected, and 80 µL of buffer C (buffer B, 0.001 mmol/L sodium pyrophosphate, 0.1% bovine serum albumin, 1 mmol/L sodium pyruvate, 0.005 mmol/L palmitoyl-L-carnitine, 10 mmol/L alpha-ketoglutaric acid, 1 mmol/L maleic acid) containing or not containing 50 µmol/L of atractyloside was added. Furthermore, buffer C (10 µL) containing 0.3 mmol/L of ADP was added to start the ATP production reaction. After reaction for 20 min, 100 µL of ATP measurement reagent (CellTiter-GloTM Luminescent Cell Viability Assay, Promega, G7571) was added to the reaction mixture to quench the reaction, and the ATP production amount was measured. The ATP production amount decreased due to the atractyloside addition was estimated as the atractyloside sensitive mitochondrial ATP production activity, which was amended based on the protein amount of the total liver homogenate measured separately, to calculate the atractyloside sensitive mitochondrial ATP production per tissue.

Table 3 shows the ferrochelatase activity, cytochrome c oxidase activity and atractyloside sensitive mitochondrial ATP production activity of the crude mitochondrial fraction of the Wistar Fatty rat- or Lean rat-derived liver. The numerical values in the Table show mean±standard deviation of n=5 - 6 (n is the number of rats).

**Table 3**

| | Lean | Fatty |
|---|---|---|
| ferrochelatase activity (Em 590 nm/mg protein) | 18.42 ± 2.59 | 11.59 ± 0.86# |
| | | |
| cytochrome c oxidase activity (units/mg protein) | 220.24 ± 13.32 | 185.04 ± 17.05 |
| | | |
| mitochondrial ATP production activity (nmol ATP/mg protein) | 0.0264 ± 0.0025 | 0.0163 ± 0.0046# |

| | | |
|---|---|---|
| #: significant difference by t-test (p<0.05). | | |

As is clear from Table 3, the crude mitochondrial fraction of the liver derived from Wistar Fatty rat showed a significant decrease in the ferrochelatase activity and atractyloside sensitive mitochondrial ATP production activity. In addition, the cytochrome c oxidase activity also showed a tendency toward a decrease. The above results suggests a possibility that the mitochondrial function decreases in Wistar Fatty rats, a decrease in the ferrochelatase activity is involved in the decreased mitochondrial function, which then leads to the onset of diabetes in Wistar Fatty rats.

### Example 3: Evaluation of ferrochelatase (FeCh) activity (A. Preparation of human-derived ferrochelatase)

### (A1. Construction of expression vector encoding mature region of human Ferrochelatase (FeCh))

Using human FeCh gene (Invitrogen) as a template, PCR was performed using the following primer 1 (SEQ ID NO: 3) and primer 2 (SEQ ID NO: 4). Pyrobest DNA polymerase (TAKARA SHUZO CO., LTD.) was used for the PCR, and extension reactions were performed under the conditions of (1) 95°C 1 min, then (2) 30 cycles of 96°C 15 sec, 60°C 30 sec, 72°C 90 sec, then (3) 72°C for 7 min. After the reaction, the amplification product was inserted into pCR-Blunt II-TOPO (Invitrogen) and cloned. The obtained plasmid was digested with Bgl II and Xho I to recover inserted fragments. The fragments were inserted into the Bam HI/Xho I site of pET43.1a (Novagen) to give an expression vector pET43.1a-hFeCh encoding a protein with Nus-Tag and His-Tag attached to the N terminal of the human FeCh Mature region (55th-423rd amino acid residues).
primer 1 (SEQ ID NO: 3)
   5'- AATTTAGATCTggtgcaaaacctcaagttcaaccg -3'
primer 2 (SEQ ID NO: 4)
   5'- AATAACTCGAGTCACAGCTGCTGGCTGGTGAAGAA -3'

### (A2. Expression of Nus-His-hFeCh (55-423) protein in Escherichia coli)

The expression vector pET43.1a-hFeCh constructed in the above-mentioned A1 was introduced into Escherichia coli BL21 (DE3) Competent Cell (Stratagene) to give an expression strain. This was precultured in LB medium (200 ml) containing Ampicilin (50 µg/ml) at 30°C overnight, and inoculated to M9 medium (3 L) containing 50 µg/ml Ampicilin and 15 g/L Casamino acid. The medium after inoculation was cultured in a jar culture apparatus DEX-2562 (Able) at 37°C, 400 rpm with aeration to reach Klett 500 KU, IPTG (Isopropyl-β-D-thiogalactopyranoside) was added to a final concentration of 1 mM and the induction culture was performed at 25°C for 3 hr. The culture was centrifuged at 6000 rpm for 15 min to collect the cells, which were preserved at -80°C.

### (A3. Purification of human FeCh)

The cells obtained in the above-mentioned A2 were suspended in Blue column Buffer (10 mM Tris-HCl (pH 8.1), 0.5 M NaCl, 10% Glycerol, 1% Triton X-100, 5 mM 2-Mercaptoethanol (2-ME), 250 ml) containing Protease Cocktail (Complete, EDTA-free; Roche). The suspension was ultrasonicated (Insonator 201M; KUBOTA CORPORATION) for 3 min (30 sec: ON, 30 sec: OFF), centrifuged at 12000 rpm for 20 min, and (NH₄)₂SO₄ was dissolved in the supernatant to 40% saturation. The obtained solution was centrifuged at 12000 rpm for 20 min, and the precipitate was dissolved in Blue column Buffer (50 ml), and dialyzed against Blue column Buffer (1 L) overnight. For purification with Blue Toyopearl, AKTAprime (Pharmacia Bioscience) was used. Toyopearl AF-Blue (Tosoh, 30 ml) was filled in a column, equilibrated with 150 ml of Blue column Buffer, and the dialyzed sample was applied. After washing with 150 ml of Blue column Buffer, the adsorbed product was eluted with 10 mM Tris-HCl (pH 8.1), 1.5 M NaCl, 10% Glycerol, 1% Sodium Cholate, 5 mM 2-ME. The eluted fractions were collected, concentrated by ultrafiltration (VivaSpin-20; Vivascience) to 9 ml, reacted overnight at room temperature using Biotinylated Thrombin (Novagen) to cleave Nus-His-Tag. After the tag cleavage, Streptavidin-Agarose (Novagen) was added, and the mixture was stirred for 30 min and passed though an empty column to remove Biotinylated Thrombin. Then, the cleaved Tag was removed using HiTrap Chelating HP (5 ml; Amersham). Ni was bound using 5 ml of 0.1 M NiSO₄, the column was equilibrated with 50 ml of Binding Buffer (10 mM Tris-HCl (pH 7.4), 0.5 M NaCl, 10% Glycerol, 1% Triton X-100, 10 mM Imidazole, 5 mM 2-Mercaptoethanol), and the sample was applied and the column was further washed with Binding Buffer. Through fraction and wash fraction were collected, concentrated by ultrafiltration (VivaSpin-20; Vivascience) to 10 ml, and the buffer was exchanged to the final buffer (10 mM Tris-HCl (pH 8.1), 150 mM NaCl, 10% Glycerol, 1% Triton X-100, 5 mM 2-ME) using NAP-25 (Amersham). As a result of protein quantification (CBB method; Pierce), the total protein amount of about 8 mg of human FeCh was obtained. After dilution of purified human FeCh protein (total protein amount 1.15 µg) with an equivalent amount of SDS-Sample Buffer (containing 2-ME; Daiichi Pure Chemicals), the mixture was heated at 95°C for 3 min, applied to SDS-PAGE (10-20%; DRC) and stained with Coomassie. From the results of SDS-PAGE, the purity was estimated to be about 70%.

### (B. Measurement of ferrochelatase activity)

The ferrochelatase activity was measured by reference to the method of RONG GUO et al. (the above-mentioned). The human recombinant ferrochelatase prepared in the above-mentioned A was diluted 500-fold with Tris-HCl buffer (pH 7.6) containing 0.01% Tween 80 and 100 nmol/L zinc acetate (pH 7.6) and added to a 96 well plate (fluoro B plate; Dainippon Pharmaceutical Co., Ltd.) for fluorescence measurement at 160 µL/well. Each of compound (a) and compound (l) was adjusted to 30 µmol/L with the above-mentioned buffer, and added to each well at 20 µL/well. 1 µmol/L of protoporphyrin IX was added to the well plate at 20 µL/well to start the reaction. The production of zinc-protoporphyrin IX was measured immediately after the start of the reaction and over time by determining at an excitation wavelength of 405 nm and a fluorescence wavelength of 590 nm. In addition, the same experiment as the above was performed without using compound (a) and compound (l), and used as the control group. The results are shown in Table 4. The numerical values in the Table show mean±standard deviation of n=8 (n is the number of measurements).

**Table 4**

| | ferrochelatase activity (Em 590 nm) |
|---|---|
| control group | 0.377±0.010 |
| compound (a) (3 µM) | 0.609±0.008 ## |
| compound (1) (3 µM) | 0.655±0.009 ## |

| | |
|---|---|
| ## : significant difference by t-test (p<0.01). | |

As is clear from Table 4, it was shown that compound (a) and compound (1) have a FeCh activation capability.

### Example 4: Evaluation of cytochrome c oxidase activity in high glucose treated HepG2 cell

(1) Human liver cancer cell-derived cell line HepG2 was passaged in low glucose (1 g/L) Eagle Modified Dulbecco's Medium supplemented with 10% fetal bovine serum. The cells adjusted to 3×10⁵/mL using the same medium were plated on a 96 well plate and precultured overnight. The aforementioned medium was changed to high glucose (4.5 g/L) Eagle Modified Dulbecco's Medium supplemented with 5 µg/mL transferrin, compound (a) or compound (l) was added and the cell culture was continued for 3 days. The same experiment was performed without using compound (a) and compound (l) as the control group.
(2) The medium was discarded, cell homogenization buffer (20 mmol/L Tris-HCl buffer pH 7.6, 0.25 mol/L sucrose, 0.5 mg/mL n-dodecyl-β-D-maltoside) was added at 120 µL/well to the well plate, and the mixture was ultrasonicated to give a cell homogenate.
(3) The DNA content was measured using PicoGreen dsDNA Quantitation Kit (Molecular Probes P-7589), and used as an index of cell number. The cytochrome c oxidase activity was determined using the Cytochrome c oxidase Assay kit (Sigma CYTOC-OX1) modified to fit a 96 well plate protocol. The aforementioned cell homogenate (40 µL) was added to an assay buffer (20 mmol/L Tris-HCl buffer pH 7.6, 120 mmol/L potassium chloride) (185 µL), and equilibrated by vortex. Reduced cytochrome c (0.4 mM, 25 µL) was added to start the reaction. The time-course changes in the absorbance were measured by determining the absorbance at 550 nm at a delta soft kinetic mode once in 15 sec for 6 min from immediately after the start of the reaction. The cytochrome c oxidase activity was calculated as an absorbance change rate in 5 min from 1 to 6 min. The cytochrome c oxidase activity of each well was amended by the content of DNA in the cell homogenate.

Table 5 and Table 6 show the cytochrome c oxidase activity of high glucose-treated HepG2 cell treated with a compound. The numerical values in the Tables show mean±standard deviation of n=4 (n is the number of wells treated with the compound).

**Table 5**

| | Cytochrome c oxidase activity (units/µg DNA) |
|---|---|
| control group | 0.649+0.044 |
| compound (a) (3 µM) | 0.757±0.029 * |

| | |
|---|---|
| *: significant difference by Williams test (p≤0.025). | |

**Table 6**

| | Cytochrome c oxidase activity (units/µg DNA) |
|---|---|
| control group | 0.743+0.043 |
| compound (l) (3 µM) | 0.990±0.088 * |

| | |
|---|---|
| *: significant difference by Williams test (p≤0.025). | |

As is clear from Table 5 and Table 6, compound (a) and compound (l) increased the cytochrome c oxidase activity of HepG2 cell. The above results show that a compound having a ferrochelatase activation capability activates cytochrome c oxidase.

### Example 5: Evaluation of anti-diabetic action in Wistar Fatty rats

Lean rats in the same lot as the male Wistar Fatty rats (26 to 27-week-old; TAKEDA RABICS, lot0402) were used. Blood samples were collected from Wistar Fatty rats in the morning under non-fasting, and glycosylated hemoglobin % value (GHB%) and plasma component were measured. GHB% was measured by Tosoh automatic glycohemoglobin analyzer HLC-723GHbV Alc2.2. The Plasma component was measured by Beckman Synchron system CX5 delta. The rats showing a GHB% value of not less than 7.5% and a high PG value were divided into 2 groups. For one group, a suspension of compound (1), obtained by suspending compound (1) in 0.5% aqueous methylcellulose solution, (dose of compound (l): 10 mg/kg body weight) was orally administered repeatedly once a day for 20 days. For the other group, 0.5% aqueous methylcellulose solution was orally administered in the same manner, and used as the control group. On the day of completion of the administration, the blood samples were collected again, and GHB% was measured. The results are shown in Table 7. The numerical values in the Table show mean±standard deviation of n=5 - 6 (n is the number of rats).

**Table 7**

| | Glycosylated hemoglobin value (%) | | |
|---|---|---|---|
| | before drug administration | after drug administration | amount of change |
| control group | 8.3 ± 0.3 | 7.7 ± 0.2 | -0.5 ± 0.2 |
| compound (l) (10 mg/kg) | 8.2 ± 0.5 | 6.9 ± 0.4 * | -1.3 ± 0.3 * |

| | | | |
|---|---|---|---|
| *: significant difference by Williams test (p≤0.025). | | | |

As is clear from Table 7, compound (l) shows a clear action to decrease GHb % value and it was confirmed that a compound having an FeCh activating action has a superior anti-diabetic action.

### Example 6: Evaluation of anti-diabetic action in ZDF rat

Male ZDF rats (5-week-old, Charles River Japan) were reared while allowing free-feeding of both diet and water. As the feed, a standard laboratory diet (MF; Oriental Yeast Co, Tokyo, Japan) and a mixed diet (the aforementioned standard laboratory diet containing 0.03% of compound (a)) were used. After acclimation rearing for 1 week, the body weight and glycated hemoglobin were measured, and the ZDF rats were divided into 2 groups (8 rats/group) such that the measurement values became uniform at 8 weeks of age. For the control group, the aforementioned standard laboratory diet was given, and for the compound (a) administration group, the aforementioned mixed diet was given from 8 weeks of age to 19 weeks of age.

At 16 weeks of age, the food intake in 24 hr was measured. The rats were reared at 4 rats/cage, the total amount of decrease in the diet in a can set to each cage was divided by 4 and taken as the daily food intake per rat. In addition, the dose of compound (a) was calculated from the food intake and the content of compound (a) in the mixed diet.

At 16 weeks of age, moreover, the blood was drawn from the rat tail vein using heparin as an anticoagulant. The blood sample was preserved on ice, and the plasma after centrifugation was preserved at -20°C until measurement. The blood glucose level was measured by a biochemical automatic analyzer (Beckman Coulter, Shyncron CX5Δ).

As a result, the blood glucose level (mean±standard deviation) of the ZDF rat at 16 weeks of age was 652.8±23.10 mg/dL for the control group, and 271.7±36.5 mg/dL (P <0.001) for the compound (a) administration group (dose: 20.9 mg/kg body weight). The compound (a) administration group showed significantly lower blood glucose levels than the control group.

From the above results, it was confirmed that a compound having an FeCh activating action has a superior anti-diabetic action.

### Example 7: Evaluation of hemoglobin increasing action

(1) After acclimation rearing for 1 week, female Crj: CD(SD)IGS rats (5-week-old) were divided into 2 groups (10 rats/group).
(2) For one group, a suspension of compound (a), obtained by suspending compound (a) in 0.5% aqueous methylcellulose solution, was orally administered at 10 ml/kg body weight/day (dose of compound (a): 600 mg/kg body weight/day) once a day. For the other group, 0.5% aqueous methylcellulose solution was orally administered at 10 ml/kg body weight/day in the same manner, and used as the control group.
(3) After 4-week administration under free-feeding conditions, the rats were fasted for 20 hr, and the whole blood was collected from the abdominal artery into a blood collection vessel (SP-U0501EM, Sekisui Chemical), and the red blood cell count, hematocrit, hemoglobin concentration, and MCH (average red blood cell hemoglobin) of the rat blood were measured. The results are shown in Table 8. The numerical values in the Table show mean±standard deviation.

**Table 8**

| | red blood cell count (x10⁴/µl) | hematocrit (%) | hemoglobin (g/dL) | MCH (pg) |
|---|---|---|---|---|
| control group | 777±21 | 42.5±1.1 | 15.3±0.4 | 19.6±0.4 |
| compound (a) (600 mg/kg/day) | 779±32 | 44.7±1.9* | 16.1±0.7* | 20.7±0.5* |

| | | | | |
|---|---|---|---|---|
| *: significant difference by Williams test (p≤0.025). | | | | |

As is clear from Table 8, the compound (a) administration group showed a significant increase in the hematocrit, hemoglobin concentration and MCH. No significant increase was observed in the red blood cell count. The above results show a possibility that compound (a) activated ferrochelatase in hematopoietic tissues and activated hem synthesis.

### Industrial Applicability

Using the screening method of the present invention, a pharmaceutical agent for the prophylaxis or treatment of diabetes or nerve system diseases caused by abnormal mitochondria can be developed. In addition, the substance obtained by the screening method of the present invention can also be used as a ferrochelatase activator as an agent for the prophylaxis or treatment of not only diabetes and nerve system diseases but also other diseases associated with decreased mitochondrial function in a tissue (e.g., mitochondrial encephalomyopathy, fibromyalgia, chronic fatigue syndrome), and disorder of organ with decreased mitochondrial function of tissue (e.g., pancreatic exhaustion, steatohepatitis). Furthermore, the substance obtained by the screening method of the present invention can be used as a ferrochelatase activator as an agent for the prophylaxis or treatment of diseases caused by decreased hem (e.g., anemia).

This application is based on a patent application No. 2006-197483 filed in Japan (filing date: July 19, 2006), the contents of which are incorporated in full herein by this reference. In addition, all publications (including patent documents) described in the present specification are hereby incorporated in their entireties by reference.

### Sequence Listing

PCT_screening method _20070718_093100_12.txt

## Claims

1. A method of screening for a therapeutic drug for diabetes or a nerve system disease, comprising using ferrochelatase.

2. The screening method of claim 1, comprising selecting a substance that activates ferrochelatase.

3. A ferrochelatase activator comprising a compound represented by the formula: 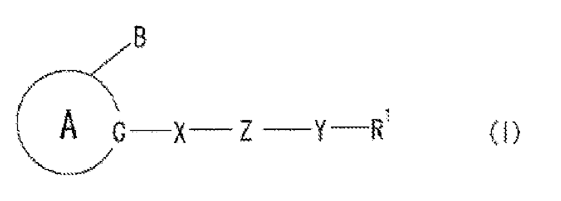 wherein ring A is a 5-membered aromatic heterocycle containing two or more nitrogen atoms and optionally further having substituent(s);
B is an optionally substituted hydrocarbon group or an optionally substituted heterocyclic group;
X is a divalent noncyclic hydrocarbon group;
Z is -O-, -S-, -NR²-, -CONR²- or -NR²CO- wherein R² is a hydrogen atom or an optionally substituted alkyl group;
Y is a bond or a divalent noncyclic hydrocarbon group; and
R¹ is an optionally substituted cyclic group, an optionally substituted amino group or an optionally substituted acyl group, or a salt thereof.

4. An agent for the prophylaxis or treatment of diabetes or a nerve system disease, which is associated with a decreased mitochondrial function in a tissue, mitochondrial encephalomyopathy, fibromyalgia, pancreatic exhaustion or steatohepatitis, comprising a ferrochelatase activator.

5. An agent for the prophylaxis or treatment of anemia, comprising a ferrochelatase activator.

6. A method for the prophylaxis or treatment of diabetes or a nerve system disease, which is associated with a decreased mitochondrial function in a tissue, mitochondrial encephalomyopathy, fibromyalgia, pancreatic exhaustion or steatohepatitis in a mammal, comprising administering an effective amount of a ferrochelatase activator to the mammal.

7. A method for the prophylaxis or treatment of anemia in a mammal, comprising administering an effective amount of a ferrochelatase activator to the mammal.

8. Use of a ferrochelatase activator for the production of an agent for the prophylaxis or treatment of diabetes or a nerve system disease, which is associated with a decreased mitochondrial function in a tissue, mitochondrial encephalomyopathy, fibromyalgia, pancreatic exhaustion or steatohepatitis.

9. Use of a ferrochelatase activator for the production of an agent for the prophylaxis or treatment of anemia.


SEQUENCE LISTING

<110>  Takeda Pharmaceutical Company Limited

<120>  Screening Method

<130>  091099

<150>  JP 2006-197483
<151>  2006-07-19

<160>  4

<170>  PatentIn version 3.3

<210>  1
<211>  1272
<212>  DNA
<213>  Homo sapiens


<220>
<221>  CDS
<222>  (1)..(1272)

<400>  1
atg cgt tca ctc ggc gca aac atg gct gcg gcc ctg cgc gcc gcg ggc       48
gtc ctg ctc cgc gat ccg ctg gca tcc agc agc tgg agg gtc tgt cag       96
cca tgg agg tgg aag tca ggt gca gct gca gcg gcc gtc acc aca gaa      144
aca gcc cag cat gcc cag ggt gca aaa cct caa gtt caa ccg cag aag      192
agg aag ccg aaa act gga ata tta atg cta aac atg gga ggc cct gaa      240
act ctt gga gat gtt cac gac ttc ctt ctg aga ctc ttc ttg gac cga      288
gac ctc atg aca ctt cct att cag aat aag ctg gca cca ttc atc gcc      336
aaa cgc cga acc ccc aag att caa gag cag tac cgc agg att gga ggc      384
gga tcc ccc atc aag ata tgg act tcc aag cag gga gag ggc atg gtg      432
aag ctg ctg gat gaa ttg tcc ccc aac aca gcc cct cac aaa tac tat 480
att gga ttt cgg tac gtc cat cct tta aca gaa gaa gca att gaa gag 528
atg gag aga gat ggc cta gaa agg gct att gct ttc aca cag tat cca 576
cag tac agc tgc tcc acc aca ggc agc agc tta aat gcc att tac aga 624
tac tat aat caa gtg gga cgg aag ccc acg atg aag tgg agc act att 672
gac agg tgg ccc aca cat cac ctc ctc atc cag tgc ttt gca gat cat 720
att cta aag gaa ctg gac cat ttt cca ctt gag aag aga agc gag gtg 768
gtc att ctg ttt tct gct cac tca ctg ccc atg tct gtg gtc aac aga 816
ggc gac cca tat cct cag gag gta agc gcc act gtc caa aaa gtc atg 864
gaa agg ctg gag tac tgc aac ccc tac cga ctg gtg tgg caa tcc aag 912
gtt ggt cca atg ccc tgg ttg ggt cct caa aca gac gaa tct atc aaa 960
ggg ctt tgt gag agg ggg agg aag aat atc ctc ttg gtt ccg ata gca 1008
ttt acc agt gac cat att gaa acg ctg tat gag ctg gac atc gag tac 1056
tct caa gtt tta gcc aag gag tgt gga gtt gaa aac atc aga aga gct 1104
gag tct ctt aat gga aat cca ttg ttc tct aag gcc ctg gcc gac ttg 1152
gtg cat tca cac atc cag tca aac gag ctg tgt tcc aag cag ctg acc     1200
ctg agc tgt ccg ctc tgt gtc aat cct gtc tgc agg gag act aaa tcc     1248
ttc ttc acc agc cag cag ctg tga                                     1272

<210>  2
<211>  423
<212>  PRT
<213>  Homo sapiens

<400>  2


<210>  3
<211>  35
<212>  DNA
<213>  Artificial

<220>
<223>  PCR primer

<400>  3
aatttagatc tggtgcaaaa cctcaagttc aaccg                                35


<210>  4
<211>  35
<212>  DNA
<213>  Artificial

<220>
<223>  PCR primer

<400>  4
aataactcga gtcacagctg ctggctggtg aagaa                                35